# EUROPEAN PATENT APPLICATION

(11) **EP 1 854 483 A1**
(43) Date of publication of application: **14.11.2007**
(21) Application number: 06713076.5
(22) Date of filing: 31.01.2006
(51) Int. Cl.: A61K 45/00, A61K 31/185, A61K 31/198, A61K 31/4402, A61K 31/4706, A61K 33/30, A61P 1/04, A61P 3/12, A61P 5/14, A61P 7/04, A61P 7/06, A61P 11/02

(54) **AGENT FOR CONTROL OF FUNCTION OF ANTIGEN-PRESENTING CELL**

(30) Priority: 31.01.2005 JP 2005023200
(71) Applicant: Riken, Wako-shi, Saitama 351-0198 (JP)
(72) Inventor: HIRANO, Toshio c/o RIKEN Yokohama Institute, Tsurumi-ku, Yokohama-shi, Kanagawa 230-0045 (JP); KITAMURA, Hidemitsu c/o RIKEN Yokohama Institute, Tsurumi-ku, Yokohama-shi, Kanagawa 230-0045 (JP)
(74) Representative: Duckworth, Timothy John
(86) International application number: PCT/JP2006/301933
(87) International publication number: WO 2006/080582

(57) **Abstract**

The present invention provides an agent capable of controlling the function of antigen-presenting cell. The present invention provides an agent for controlling the function of antigen-presenting cell, comprising a substance capable of controlling an intracellular zinc ion concentration, particularly, a zinc ion, a zinc ion chelator, a substance for regulating the expression and/or function of a zinc ion-requiring protein, or a substance for regulating the expression and/or function of a zinc ion transporter, as an active ingredient.

SEQUENCE LISTING

<110> RIKEN

<120> AGENT FOR CONTROL OF FUNCTION OF ANTIGEN-PRESENTING CELL

<130> 09868

<150> JP 2005-023200
<151> 2005-01-31

<160> 8

<170> PatentIn version 3.3

<210> 1
<211> 2229
<212> DNA
<213> Danio rerio

<220>
<221> CDS
<222> (1)..(2229)

<400> 1
atg atg acg ttt ctt tgc aca cgg tct ggt cgc cgt gct agt ggt gtg 48
Met Met Thr Phe Leu Cys Thr Arg Ser Gly Arg Arg Ala Ser Gly Val
1               5                   10                  15

gag tgc aga atc gcc gct gaa cgc gct tac ttt cga gtg cgt gga ctc 96
Glu Cys Arg Ile Ala Ala Glu Arg Ala Tyr Phe Arg Val Arg Gly Leu
            20                  25                  30

ccg gtt gcc aat atg att ggc tgg tgg cca cgc ctc tgc cca gtg atg 144
Pro Val Ala Asn Met Ile Gly Trp Trp Pro Arg Leu Cys Pro Val Met
        35                  40                  45

tca ctg gca ctg ctg tgg gcg tgt tca gtg ggg gcg ggt tca gac tgc 192
Ser Leu Ala Leu Leu Trp Ala Cys Ser Val Gly Ala Gly Ser Asp Cys
    50                  55                  60

aaa tct gtg gcc att gag act gac agc cgc ata gca gaa caa aca cag 240
Lys Ser Val Ala Ile Glu Thr Asp Ser Arg Ile Ala Glu Gln Thr Gln
65                  70                  75                  80

cag cgt cac cta cag gct ctg ttc gac aag tat ggc cag aac ggc agc 288
Gln Arg His Leu Gln Ala Leu Phe Asp Lys Tyr Gly Gln Asn Gly Ser
                85                  90                  95

atc tcc cta gaa ggc ctc ttc aac cta ctt aaa ggg gtc ggg ctt gac 336
Ile Ser Leu Glu Gly Leu Phe Asn Leu Leu Lys Gly Val Gly Leu Asp
            100                 105                 110

cgc atc cgg aaa gtg atg gtg cat cat cct gga aat gcc cat aat cac 384
Arg Ile Arg Lys Val Met Val His His Pro Gly Asn Ala His Asn His
        115                 120                 125

aca cac acg cat gat cac aca cac act cat gtg gac aaa ctc acg gcg 432
Thr His Thr His Asp His Thr His Thr His Val Asp Lys Leu Thr Ala
    130                 135                 140

cac aca cat ccg gtc acc acc aag aag gga gac atg gat cac agc gtg 480
His Thr His Pro Val Thr Thr Lys Lys Gly Asp Met Asp His Ser Val
145                 150                 155                 160

gag aag agt gac cct gtc cca aaa gca cag cca gat cct gcc tct ggg 528
Glu Lys Ser Asp Pro Val Pro Lys Ala Gln Pro Asp Pro Ala Ser Gly
                165                 170                 175

aag aaa agc cag tca gat gcg cat cac aac ctg tac atg aag atg aac 576
Lys Lys Ser Gln Ser Asp Ala His His Asn Leu Tyr Met Lys Met Asn
            180                 185                 190

cag gaa tcc acc aca gct ttg act acg ccg tca tat gtt acc aga tca 624
Gln Glu Ser Thr Thr Ala Leu Thr Thr Pro Ser Tyr Val Thr Arg Ser

        195                 200                 205

cgg cgg acc aat cgc agc gcc gat tat gat ttt aca cag gac cac gcc 672
Arg Arg Thr Asn Arg Ser Ala Asp Tyr Asp Phe Thr Gln Asp His Ala
    210                 215                 220

tcc ttt agc ccc agt cag ccc aat gtg aca cac tca aac cac acc cat 720
Ser Phe Ser Pro Ser Gln Pro Asn Val Thr His Ser Asn His Thr His
225                 230                 235                 240

cat gat gag gac acg ccc aca cac cag cat gat gac cat gat gag cac 768
His Asp Glu Asp Thr Pro Thr His Gln His Asp Asp His Asp Glu His
                245                 250                 255

gaa cat gcc cgt gct agt tta ggg tgt caa aat gcc tcc acg atc ctg 816
Glu His Ala Arg Ala Ser Leu Gly Cys Gln Asn Ala Ser Thr Ile Leu
            260                 265                 270

cag acg cat ggc atg aga aag gaa gca agt ctc tca gtt aag gac ttc 864
Gln Thr His Gly Met Arg Lys Glu Ala Ser Leu Ser Val Lys Asp Phe
        275                 280                 285

agt ttc ctc tgc cct gct ctt ctc atg cag att gat tcc aag tct tgc 912
Ser Phe Leu Cys Pro Ala Leu Leu Met Gln Ile Asp Ser Lys Ser Cys
    290                 295                 300

atc gtg cat gaa gac gag gac gag cat tca gat cat tcc cat cat cac 960
Ile Val His Glu Asp Glu Asp Glu His Ser Asp His Ser His His His
305                 310                 315                 320

aaa cac cac cac cat cat cat gat cac caa cac ctg cag cat cca cat 1008
Lys His His His His His His Asp His Gln His Leu Gln His Pro His
                325                 330                 335

aac cac acc aat gga aga ggc cag agg aac act cca gtc tac atc gca 1056
Asn His Thr Asn Gly Arg Gly Gln Arg Asn Thr Pro Val Tyr Ile Ala
            340                 345                 350

tgg ctt gga ggg ttt ctc tcc atc act ctg atc agt ttg ctg gcg ttg 1104
Trp Leu Gly Gly Phe Leu Ser Ile Thr Leu Ile Ser Leu Leu Ala Leu
        355                 360                 365

gtt ggt gtg gtt ttg atc cca ctc atg aac aga gtt tgc ttc aac ttc 1152
Val Gly Val Val Leu Ile Pro Leu Met Asn Arg Val Cys Phe Asn Phe
    370                 375                 380

ctg ctg agc ttc ctg gtg gcc ctt gca gtg ggc act ctg agc gga gac 1200
Leu Leu Ser Phe Leu Val Ala Leu Ala Val Gly Thr Leu Ser Gly Asp
385                 390                 395                 400

gct ctc ctc cac ctc ata cca cat tct cag ggt cat cac cat cac ggc 1248
Ala Leu Leu His Leu Ile Pro His Ser Gln Gly His His His His Gly
                405                 410                 415

cac tct gaa gag cac gct gaa gag gag gac tcc ctt cgc cct gtg tgg 1296
His Ser Glu Glu His Ala Glu Glu Glu Asp Ser Leu Arg Pro Val Trp
            420                 425                 430

acc gga ctc aca gct cta agt gga gtt tac atc atg ttc ctc atc gaa 1344
Thr Gly Leu Thr Ala Leu Ser Gly Val Tyr Ile Met Phe Leu Ile Glu
        435                 440                 445

cac ttc ctg acc ctt ggc aaa atg tac aaa gac aaa aac cag aag gtg 1392
His Phe Leu Thr Leu Gly Lys Met Tyr Lys Asp Lys Asn Gln Lys Val
    450                 455                 460

cag aag agg gtt gat ctc acc aca gaa gtt ttg gag tct gag aaa ctg 1440
Gln Lys Arg Val Asp Leu Thr Thr Glu Val Leu Glu Ser Glu Lys Leu
465                 470                 475                 480

cca tca tta gaa gaa aat gat gtc aaa att gaa gct gct gaa acg aat 1488
Pro Ser Leu Glu Glu Asn Asp Val Lys Ile Glu Ala Ala Glu Thr Asn
                485                 490                 495

ggt ggg cgt gca ctg gca gag gag gag gag gtg atg ttg ggg gcg gag 1536
Gly Gly Arg Ala Leu Ala Glu Glu Glu Glu Val Met Leu Gly Ala Glu

            500                 505                 510

ctc tac aac gac ata gac tgc gag aac aaa tgc cac tcc cac ttc cat 1584
Leu Tyr Asn Asp Ile Asp Cys Glu Asn Lys Cys His Ser His Phe His
        515                 520                 525

gac acg gtc ggc caa tcg gat gag cag cat cat cat cat cac gac tac 1632
Asp Thr Val Gly Gln Ser Asp Glu Gln His His His His His Asp Tyr
    530                 535                 540

cac cac ata ctg cat cat cac cac tcc cag aac cac cac ccg cac aca 1680
His His Ile Leu His His His His Ser Gln Asn His His Pro His Thr
545                 550                 555                 560

cac acg cac aga cac aca cac tcc tac tcg cag cag cac ttt gag cag 1728
His Thr His Arg His Thr His Ser Tyr Ser Gln Gln His Phe Glu Gln
                565                 570                 575

gct ggt gtg gcc aca ctc gcc tgg atg gtc atc atg gga gac gga ctg 1776
Ala Gly Val Ala Thr Leu Ala Trp Met Val Ile Met Gly Asp Gly Leu
            580                 585                 590

cac aac ttc agt gat gga ctt gcc ata ggg gcg gct ttc aca gaa ggt 1824
His Asn Phe Ser Asp Gly Leu Ala Ile Gly Ala Ala Phe Thr Glu Gly
        595                 600                 605

ttg tcc agt ggt ctt agt acc tca gtc gct gtg ttc tgc cat gag ctt 1872
Leu Ser Ser Gly Leu Ser Thr Ser Val Ala Val Phe Cys His Glu Leu
    610                 615                 620

cct cat gaa ctc ggt gat ttt gcc gtc cta ctg aaa gcc ggt atg tca 1920
Pro His Glu Leu Gly Asp Phe Ala Val Leu Leu Lys Ala Gly Met Ser
625                 630                 635                 640

gtt cga cag gcc atg ctg tat aat ctg ctg tca gca ctg atg gga tat 1968
Val Arg Gln Ala Met Leu Tyr Asn Leu Leu Ser Ala Leu Met Gly Tyr
                645                 650                 655

ctg ggc atg atc atc ggg att ctc atc gga cat tat gct gaa aat gtt 2016
Leu Gly Met Ile Ile Gly Ile Leu Ile Gly His Tyr Ala Glu Asn Val
            660                 665                 670

gcc aca tgg atc ttt gct ctc aca gct ggg tta ttc atg tac gtc gcg 2064
Ala Thr Trp Ile Phe Ala Leu Thr Ala Gly Leu Phe Met Tyr Val Ala
        675                 680                 685

ctc gtg gac atg gta cct gag atg ctg cac aat gac gcg agc gaa gca 2112
Leu Val Asp Met Val Pro Glu Met Leu His Asn Asp Ala Ser Glu Ala
    690                 695                 700

ggt ttc agt cac tac ggc ttc ttc ctc ctg cag aac gct ggg ata ctc 2160
Gly Phe Ser His Tyr Gly Phe Phe Leu Leu Gln Asn Ala Gly Ile Leu
705                 710                 715                 720

cta ggc ttc ggc atc atg ctt atc att gct gtc ttt gag gac agg atc 2208
Leu Gly Phe Gly Ile Met Leu Ile Ile Ala Val Phe Glu Asp Arg Ile
                725                 730                 735

caa ctg gac tta ggt tac tga 2229
Gln Leu Asp Leu Gly Tyr
            740

<210> 2
<211> 742
<212> PRT
<213> Danio rerio

<400> 2

Met Met Thr Phe Leu Cys Thr Arg Ser Gly Arg Arg Ala Ser Gly Val
1               5                   10                  15

Glu Cys Arg Ile Ala Ala Glu Arg Ala Tyr Phe Arg Val Arg Gly Leu
            20                  25                  30

Pro Val Ala Asn Met Ile Gly Trp Trp Pro Arg Leu Cys Pro Val Met
        35                  40                  45

Ser Leu Ala Leu Leu Trp Ala Cys Ser Val Gly Ala Gly Ser Asp Cys
    50                  55                  60

Lys Ser Val Ala Ile Glu Thr Asp Ser Arg Ile Ala Glu Gln Thr Gln
65                  70                  75                  80

Gln Arg His Leu Gln Ala Leu Phe Asp Lys Tyr Gly Gln Asn Gly Ser
                85                  90                  95

Ile Ser Leu Glu Gly Leu Phe Asn Leu Leu Lys Gly Val Gly Leu Asp
            100                 105                 110

Arg Ile Arg Lys Val Met Val His His Pro Gly Asn Ala His Asn His
        115                 120                 125

Thr His Thr His Asp His Thr His Thr His Val Asp Lys Leu Thr Ala
    130                 135                 140

His Thr His Pro Val Thr Thr Lys Lys Gly Asp Met Asp His Ser Val
145                 150                 155                 160

Glu Lys Ser Asp Pro Val Pro Lys Ala Gln Pro Asp Pro Ala Ser Gly
                165                 170                 175

Lys Lys Ser Gln Ser Asp Ala His His Asn Leu Tyr Met Lys Met Asn
            180                 185                 190

Gln Glu Ser Thr Thr Ala Leu Thr Thr Pro Ser Tyr Val Thr Arg Ser
        195                 200                 205

Arg Arg Thr Asn Arg Ser Ala Asp Tyr Asp Phe Thr Gln Asp His Ala
    210                 215                 220

Ser Phe Ser Pro Ser Gln Pro Asn Val Thr His Ser Asn His Thr His
225                 230                 235                 240

His Asp Glu Asp Thr Pro Thr His Gln His Asp Asp His Asp Glu His
                245                 250                 255

Glu His Ala Arg Ala Ser Leu Gly Cys Gln Asn Ala Ser Thr Ile Leu
            260                 265                 270

Gln Thr His Gly Met Arg Lys Glu Ala Ser Leu Ser Val Lys Asp Phe
        275                 280                 285

Ser Phe Leu Cys Pro Ala Leu Leu Met Gln Ile Asp Ser Lys Ser Cys
    290                 295                 300

Ile Val His Glu Asp Glu Asp Glu His Ser Asp His Ser His His His
305                 310                 315                 320

Lys His His His His His His Asp His Gln His Leu Gln His Pro His
                325                 330                 335

Asn His Thr Asn Gly Arg Gly Gln Arg Asn Thr Pro Val Tyr Ile Ala
            340                 345                 350

Trp Leu Gly Gly Phe Leu Ser Ile Thr Leu Ile Ser Leu Leu Ala Leu
        355                 360                 365

Val Gly Val Val Leu Ile Pro Leu Met Asn Arg Val Cys Phe Asn Phe
    370                 375                 380

Leu Leu Ser Phe Leu Val Ala Leu Ala Val Gly Thr Leu Ser Gly Asp
385                 390                 395                 400

Ala Leu Leu His Leu Ile Pro His Ser Gln Gly His His His His Gly
                405                 410                 415

His Ser Glu Glu His Ala Glu Glu Glu Asp Ser Leu Arg Pro Val Trp
            420                 425                 430

Thr Gly Leu Thr Ala Leu Ser Gly Val Tyr Ile Met Phe Leu Ile Glu
        435                 440                 445

His Phe Leu Thr Leu Gly Lys Met Tyr Lys Asp Lys Asn Gln Lys Val
    450                 455                 460

Gln Lys Arg Val Asp Leu Thr Thr Glu Val Leu Glu Ser Glu Lys Leu
465                 470                 475                 480

Pro Ser Leu Glu Glu Asn Asp Val Lys Ile Glu Ala Ala Glu Thr Asn
                485                 490                 495

Gly Gly Arg Ala Leu Ala Glu Glu Glu Glu Val Met Leu Gly Ala Glu
            500                 505                 510

Leu Tyr Asn Asp Ile Asp Cys Glu Asn Lys Cys His Ser His Phe His
        515                 520                 525

Asp Thr Val Gly Gln Ser Asp Glu Gln His His His His His Asp Tyr
    530                 535                 540

His His Ile Leu His His His His Ser Gln Asn His His Pro His Thr
545                 550                 555                 560

His Thr His Arg His Thr His Ser Tyr Ser Gln Gln His Phe Glu Gln
                565                 570                 575

Ala Gly Val Ala Thr Leu Ala Trp Met Val Ile Met Gly Asp Gly Leu
            580                 585                 590

His Asn Phe Ser Asp Gly Leu Ala Ile Gly Ala Ala Phe Thr Glu Gly
        595                 600                 605

Leu Ser Ser Gly Leu Ser Thr Ser Val Ala Val Phe Cys His Glu Leu
    610                 615                 620

Pro His Glu Leu Gly Asp Phe Ala Val Leu Leu Lys Ala Gly Met Ser
625                 630                 635                 640

Val Arg Gln Ala Met Leu Tyr Asn Leu Leu Ser Ala Leu Met Gly Tyr
                645                 650                 655

Leu Gly Met Ile Ile Gly Ile Leu Ile Gly His Tyr Ala Glu Asn Val
            660                 665                 670

Ala Thr Trp Ile Phe Ala Leu Thr Ala Gly Leu Phe Met Tyr Val Ala
        675                 680                 685

Leu Val Asp Met Val Pro Glu Met Leu His Asn Asp Ala Ser Glu Ala
    690                 695                 700

Gly Phe Ser His Tyr Gly Phe Phe Leu Leu Gln Asn Ala Gly Ile Leu
705                 710                 715                 720

Leu Gly Phe Gly Ile Met Leu Ile Ile Ala Val Phe Glu Asp Arg Ile
                725                 730                 735

Gln Leu Asp Leu Gly Tyr
            740

<210> 3
<211> 2268
<212> DNA
<213> Homo sapiens

<220>
<221> CDS
<222> (1)..(2268)

<400> 3
atg gcg agg aag tta tct gta atc ttg atc ctg acc ttt gcc ctc tct 48
Met Ala Arg Lys Leu Ser Val Ile Leu Ile Leu Thr Phe Ala Leu Ser
1               5                   10                  15

gtc aca aat ccc ctt cat gaa cta aaa gca gct gct ttc ccc cag acc 96
Val Thr Asn Pro Leu His Glu Leu Lys Ala Ala Ala Phe Pro Gln Thr
            20                  25                  30

act gag aaa att agt ccg aat tgg gaa tct ggc att aat gtt gac ttg 144
Thr Glu Lys Ile Ser Pro Asn Trp Glu Ser Gly Ile Asn Val Asp Leu
        35                  40                  45

gca att tcc aca cgg caa tat cat cta caa cag ctt ttc tac cgc tat 192
Ala Ile Ser Thr Arg Gln Tyr His Leu Gln Gln Leu Phe Tyr Arg Tyr
    50                  55                  60

gga gaa aat aat tct ttg tca gtt gaa ggg ttc aga aaa tta ctt caa 240
Gly Glu Asn Asn Ser Leu Ser Val Glu Gly Phe Arg Lys Leu Leu Gln
65                  70                  75                  80

aat ata ggc ata gat aag att aaa aga atc cat ata cac cat gac cac 288
Asn Ile Gly Ile Asp Lys Ile Lys Arg Ile His Ile His His Asp His
                85                  90                  95

gac cat cac tca gac cac gag cat cac tca gac cat gag cgt cac tca 336
Asp His His Ser Asp His Glu His His Ser Asp His Glu Arg His Ser
            100                 105                 110

gac cat gag cat cac tca gac cac gag cat cac tct gac cat gat cat 384
Asp His Glu His His Ser Asp His Glu His His Ser Asp His Asp His
        115                 120                 125

cac tct cac cat aat cat gct gct tct ggt aaa aat aag cga aaa gct 432
His Ser His His Asn His Ala Ala Ser Gly Lys Asn Lys Arg Lys Ala
    130                 135                 140

ctt tgc cca gac cat gac tca gat agt tca ggt aaa gat cct aga aac 480
Leu Cys Pro Asp His Asp Ser Asp Ser Ser Gly Lys Asp Pro Arg Asn
145                 150                 155                 160

agc cag ggg aaa gga gct cac cga cca gaa cat gcc agt ggt aga agg 528
Ser Gln Gly Lys Gly Ala His Arg Pro Glu His Ala Ser Gly Arg Arg
                165                 170                 175

aat gtc aag gac agt gtt agt gct agt gaa gtg acc tca act gtg tac 576
Asn Val Lys Asp Ser Val Ser Ala Ser Glu Val Thr Ser Thr Val Tyr
            180                 185                 190

aac act gtc tct gaa gga act cac ttt cta gag aca ata gag act cca 624
Asn Thr Val Ser Glu Gly Thr His Phe Leu Glu Thr Ile Glu Thr Pro
        195                 200                 205

aga cct gga aaa ctc ttc ccc aaa gat gta agc agc tcc act cca ccc 672
Arg Pro Gly Lys Leu Phe Pro Lys Asp Val Ser Ser Ser Thr Pro Pro
    210                 215                 220

agt gtc aca tca aag agc cgg gtg agc cgg ctg gct ggt agg aaa aca 720
Ser Val Thr Ser Lys Ser Arg Val Ser Arg Leu Ala Gly Arg Lys Thr
225                 230                 235                 240

aat gaa tct gtg agt gag ccc cga aaa ggc ttt atg tat tcc aga aac 768
Asn Glu Ser Val Ser Glu Pro Arg Lys Gly Phe Met Tyr Ser Arg Asn
                245                 250                 255

aca aat gaa aat cct cag gag tgt ttc aat gca tca aag cta ctg aca 816
Thr Asn Glu Asn Pro Gln Glu Cys Phe Asn Ala Ser Lys Leu Leu Thr
            260                 265                 270

tct cat ggc atg ggc atc cag gtt ccg ctg aat gca aca gag ttc aac 864
Ser His Gly Met Gly Ile Gln Val Pro Leu Asn Ala Thr Glu Phe Asn
        275                 280                 285

tat ctc tgt cca gcc atc atc aac caa att gat gct aga tct tgt ctg 912
Tyr Leu Cys Pro Ala Ile Ile Asn Gln Ile Asp Ala Arg Ser Cys Leu
    290                 295                 300

att cat aca agt gaa aag aag gct gaa atc cct cca aag acc tat tca 960
Ile His Thr Ser Glu Lys Lys Ala Glu Ile Pro Pro Lys Thr Tyr Ser
305                 310                 315                 320

tta caa ata gcc tgg gtt ggt ggt ttt ata gcc att tcc atc atc agt 1008
Leu Gln Ile Ala Trp Val Gly Gly Phe Ile Ala Ile Ser Ile Ile Ser
                325                 330                 335

ttc ctg tct ctg ctg ggg gtt atc tta gtg cct ctc atg aat cgg gtg 1056
Phe Leu Ser Leu Leu Gly Val Ile Leu Val Pro Leu Met Asn Arg Val
            340                 345                 350

ttt ttc aaa ttt ctc ctg agt ttc ctt gtg gca ctg gcc gtt ggg act 1104
Phe Phe Lys Phe Leu Leu Ser Phe Leu Val Ala Leu Ala Val Gly Thr
        355                 360                 365

ttg agt ggt gat gct ttt tta cac ctt ctt cca cat tct cat gca agt 1152
Leu Ser Gly Asp Ala Phe Leu His Leu Leu Pro His Ser His Ala Ser
    370                 375                 380

cac cac cat agt cat agc cat gaa gaa cca gca atg gaa atg aaa aga 1200
His His His Ser His Ser His Glu Glu Pro Ala Met Glu Met Lys Arg
385                 390                 395                 400

gga cca ctt ttc agt cat ctg tct tct caa aac ata gaa gaa agt gcc 1248
Gly Pro Leu Phe Ser His Leu Ser Ser Gln Asn Ile Glu Glu Ser Ala
                405                 410                 415

tat ttt gat tcc acg tgg aag ggt cta aca gct cta gga ggc ctg tat 1296
Tyr Phe Asp Ser Thr Trp Lys Gly Leu Thr Ala Leu Gly Gly Leu Tyr
            420                 425                 430

ttc atg ttt ctt gtt gaa cat gtc ctc aca ttg atc aaa caa ttt aaa 1344
Phe Met Phe Leu Val Glu His Val Leu Thr Leu Ile Lys Gln Phe Lys
        435                 440                 445

gat aag aag aaa aag aat cag aag aaa cct gaa aat gat gat gat gtg     1392
Asp Lys Lys Lys Lys Asn Gln Lys Lys Pro Glu Asn Asp Asp Asp Val
    450                 455                 460

gag att aag aag cag ttg tcc aag tat gaa tct caa ctt tca aca aat     1440
Glu Ile Lys Lys Gln Leu Ser Lys Tyr Glu Ser Gln Leu Ser Thr Asn
465                 470                 475                 480

gag gag aaa gta gat aca gat gat cga act gaa ggc tat tta cga gca     1488
Glu Glu Lys Val Asp Thr Asp Asp Arg Thr Glu Gly Tyr Leu Arg Ala
                485                 490                 495

gac tca caa gag ccc tcc cac ttt gat tct cag cag cct gca gtc ttg     1536
Asp Ser Gln Glu Pro Ser His Phe Asp Ser Gln Gln Pro Ala Val Leu
            500                 505                 510

gaa gaa gaa gag gtc atg ata gct cat gct cat cca cag gaa gtc tac     1584
Glu Glu Glu Glu Val Met Ile Ala His Ala His Pro Gln Glu Val Tyr
        515                 520                 525

aat gaa tat gta ccc aga ggg tgc aag aat aaa tgc cat tca cat ttc     1632
Asn Glu Tyr Val Pro Arg Gly Cys Lys Asn Lys Cys His Ser His Phe
    530                 535                 540

cac gat aca ctc ggc cag tca gac gat ctc att cac cac cat cat gac     1680
His Asp Thr Leu Gly Gln Ser Asp Asp Leu Ile His His His His Asp
545                 550                 555                 560

tac cat cat att ctc cat cat cac cac cac caa aac cac cat cct cac     1728
Tyr His His Ile Leu His His His His His Gln Asn His His Pro His
                565                 570                 575

agt cac agc cag cgc tac tct cgg gag gag ctg aaa gat gcc ggc gtc     1776
Ser His Ser Gln Arg Tyr Ser Arg Glu Glu Leu Lys Asp Ala Gly Val
            580                 585                 590

gcc act ttg gcc tgg atg gtg ata atg ggt gat ggc ctg cac aat ttc     1824
Ala Thr Leu Ala Trp Met Val Ile Met Gly Asp Gly Leu His Asn Phe
        595                 600                 605

agc gat ggc cta gca att ggt gct gct ttt act gaa ggc tta tca agt     1872
Ser Asp Gly Leu Ala Ile Gly Ala Ala Phe Thr Glu Gly Leu Ser Ser
    610                 615                 620

ggt tta agt act tct gtt gct gtg ttc tgt cat gag ttg cct cat gaa     1920
Gly Leu Ser Thr Ser Val Ala Val Phe Cys His Glu Leu Pro His Glu
625                 630                 635                 640

tta ggt gac ttt gct gtt cta cta aag gct ggc atg acc gtt aag cag     1968
Leu Gly Asp Phe Ala Val Leu Leu Lys Ala Gly Met Thr Val Lys Gln
                645                 650                 655

gct gtc ctt tat aat gca ttg tca gcc atg ctg gcg tat ctt gga atg     2016
Ala Val Leu Tyr Asn Ala Leu Ser Ala Met Leu Ala Tyr Leu Gly Met
            660                 665                 670

gca aca gga att ttc att ggt cat tat gct gaa aat gtt tct atg tgg     2064
Ala Thr Gly Ile Phe Ile Gly His Tyr Ala Glu Asn Val Ser Met Trp
        675                 680                 685

ata ttt gca ctt act gct ggc tta ttc atg tat gtt gct ctg gtt gat     2112
Ile Phe Ala Leu Thr Ala Gly Leu Phe Met Tyr Val Ala Leu Val Asp
    690                 695                 700

atg gta cct gaa atg ctg cac aat gat gct agt gac cat gga tgt agc     2160
Met Val Pro Glu Met Leu His Asn Asp Ala Ser Asp His Gly Cys Ser
705                 710                 715                 720

cgc tgg ggg tat ttc ttt tta cag aat gct ggg atg ctt ttg ggt ttt     2208
Arg Trp Gly Tyr Phe Phe Leu Gln Asn Ala Gly Met Leu Leu Gly Phe
                725                 730                 735

gga att atg tta ctt att tcc ata ttt gaa cat aaa atc gtg ttt cgt     2256
Gly Ile Met Leu Leu Ile Ser Ile Phe Glu His Lys Ile Val Phe Arg
            740                 745                 750

ata aat ttc tag                                                    2268
Ile Asn Phe
        755


<210>  4
<211>  755
<212>  PRT
<213>  Homo sapiens

<400>  4

Met Ala Arg Lys Leu Ser Val Ile Leu Ile Leu Thr Phe Ala Leu Ser
1               5                   10                  15

Val Thr Asn Pro Leu His Glu Leu Lys Ala Ala Ala Phe Pro Gln Thr
            20                  25                  30

Thr Glu Lys Ile Ser Pro Asn Trp Glu Ser Gly Ile Asn Val Asp Leu
        35                  40                  45

Ala Ile Ser Thr Arg Gln Tyr His Leu Gln Gln Leu Phe Tyr Arg Tyr
    50                  55                  60

Gly Glu Asn Asn Ser Leu Ser Val Glu Gly Phe Arg Lys Leu Leu Gln
65                  70                  75                  80

Asn Ile Gly Ile Asp Lys Ile Lys Arg Ile His Ile His His Asp His
                85                  90                  95

Asp His His Ser Asp His Glu His His Ser Asp His Glu Arg His Ser
            100                 105                 110

Asp His Glu His His Ser Asp His Glu His His Ser Asp His Asp His
        115                 120                 125

His Ser His His Asn His Ala Ala Ser Gly Lys Asn Lys Arg Lys Ala
    130                 135                 140

Leu Cys Pro Asp His Asp Ser Asp Ser Ser Gly Lys Asp Pro Arg Asn
145                 150                 155                 160

Ser Gln Gly Lys Gly Ala His Arg Pro Glu His Ala Ser Gly Arg Arg
                165                 170                 175

Asn Val Lys Asp Ser Val Ser Ala Ser Glu Val Thr Ser Thr Val Tyr
            180                 185                 190

Asn Thr Val Ser Glu Gly Thr His Phe Leu Glu Thr Ile Glu Thr Pro
        195                 200                 205

Arg Pro Gly Lys Leu Phe Pro Lys Asp Val Ser Ser Ser Thr Pro Pro
    210                 215                 220

Ser Val Thr Ser Lys Ser Arg Val Ser Arg Leu Ala Gly Arg Lys Thr
225                 230                 235                 240

Asn Glu Ser Val Ser Glu Pro Arg Lys Gly Phe Met Tyr Ser Arg Asn
                245                 250                 255

Thr Asn Glu Asn Pro Gln Glu Cys Phe Asn Ala Ser Lys Leu Leu Thr
            260                     265                     270

Ser His Gly Met Gly Ile Gln Val Pro Leu Asn Ala Thr Glu Phe Asn
        275                     280                     285

Tyr Leu Cys Pro Ala Ile Ile Asn Gln Ile Asp Ala Arg Ser Cys Leu
    290                     295                     300

Ile His Thr Ser Glu Lys Lys Ala Glu Ile Pro Pro Lys Thr Tyr Ser
305                     310                     315                     320

Leu Gln Ile Ala Trp Val Gly Gly Phe Ile Ala Ile Ser Ile Ile Ser
                325                     330                     335

Phe Leu Ser Leu Leu Gly Val Ile Leu Val Pro Leu Met Asn Arg Val
            340                     345                     350

Phe Phe Lys Phe Leu Leu Ser Phe Leu Val Ala Leu Ala Val Gly Thr
        355                     360                     365

Leu Ser Gly Asp Ala Phe Leu His Leu Leu Pro His Ser His Ala Ser
    370                     375                     380

His His His Ser His Ser His Glu Glu Pro Ala Met Glu Met Lys Arg
385                     390                     395                     400

Gly Pro Leu Phe Ser His Leu Ser Ser Gln Asn Ile Glu Glu Ser Ala
                405                     410                     415

Tyr Phe Asp Ser Thr Trp Lys Gly Leu Thr Ala Leu Gly Gly Leu Tyr
            420                     425                     430

Phe Met Phe Leu Val Glu His Val Leu Thr Leu Ile Lys Gln Phe Lys
        435                     440                     445

Asp Lys Lys Lys Lys Asn Gln Lys Lys Pro Glu Asn Asp Asp Asp Val
    450                     455                     460

Glu Ile Lys Lys Gln Leu Ser Lys Tyr Glu Ser Gln Leu Ser Thr Asn
465                     470                     475                     480

Glu Glu Lys Val Asp Thr Asp Asp Arg Thr Glu Gly Tyr Leu Arg Ala
                485                     490                     495

Asp Ser Gln Glu Pro Ser His Phe Asp Ser Gln Gln Pro Ala Val Leu
            500                     505                     510

Glu Glu Glu Glu Val Met Ile Ala His Ala His Pro Gln Glu Val Tyr
        515                     520                     525

Asn Glu Tyr Val Pro Arg Gly Cys Lys Asn Lys Cys His Ser His Phe
    530                     535                     540

His Asp Thr Leu Gly Gln Ser Asp Asp Leu Ile His His His His Asp
545                     550                     555                     560

Tyr His His Ile Leu His His His His His Gln Asn His His Pro His
                565                 570                 575

Ser His Ser Gln Arg Tyr Ser Arg Glu Glu Leu Lys Asp Ala Gly Val
            580                 585                 590

Ala Thr Leu Ala Trp Met Val Ile Met Gly Asp Gly Leu His Asn Phe
        595                 600                 605

Ser Asp Gly Leu Ala Ile Gly Ala Ala Phe Thr Glu Gly Leu Ser Ser
    610                 615                 620

Gly Leu Ser Thr Ser Val Ala Val Phe Cys His Glu Leu Pro His Glu
625                 630                 635                 640

Leu Gly Asp Phe Ala Val Leu Leu Lys Ala Gly Met Thr Val Lys Gln
                645                 650                 655

Ala Val Leu Tyr Asn Ala Leu Ser Ala Met Leu Ala Tyr Leu Gly Met
            660                 665                 670

Ala Thr Gly Ile Phe Ile Gly His Tyr Ala Glu Asn Val Ser Met Trp
        675                 680                 685

Ile Phe Ala Leu Thr Ala Gly Leu Phe Met Tyr Val Ala Leu Val Asp
    690                 695                 700

Met Val Pro Glu Met Leu His Asn Asp Ala Ser Asp His Gly Cys Ser
705                 710                 715                 720

Arg Trp Gly Tyr Phe Phe Leu Gln Asn Ala Gly Met Leu Leu Gly Phe
                725                 730                 735

Gly Ile Met Leu Leu Ile Ser Ile Phe Glu His Lys Ile Val Phe Arg
            740                 745                 750

Ile Asn Phe
        755

<210>  5
<211>  2298
<212>  DNA
<213>  Mus musculus

<220>
<221>  CDS
<222>  (1)..(2298)

<400>  5
atg gcc aca gat tta tct gta atc atg atc ttg acc ttt gcc ctt tgg        48
Met Ala Thr Asp Leu Ser Val Ile Met Ile Leu Thr Phe Ala Leu Trp
1               5                   10                  15

gtt aca agc ccc ctt cat gaa cta caa tca aca gct gct ttc tct cag        96
Val Thr Ser Pro Leu His Glu Leu Gln Ser Thr Ala Ala Phe Ser Gln
            20                  25                  30

act act gag aaa ata aat tca aat tgg gaa cct ggt gtt aat gtt gac       144
Thr Thr Glu Lys Ile Asn Ser Asn Trp Glu Pro Gly Val Asn Val Asp
        35                  40                  45

ttg gca gtt acc atg cag cga cac cat ctg cag cag cta ttc tac cgc 192
Leu Ala Val Thr Met Gln Arg His His Leu Gln Gln Leu Phe Tyr Arg
    50                  55                  60

tac gga gag aat gat tcc ttg tct gtt gaa ggc ttc aga aaa ttg ctt 240
Tyr Gly Glu Asn Asp Ser Leu Ser Val Glu Gly Phe Arg Lys Leu Leu
65                  70                  75                  80

cag aac ata ggc ata gat aag att aaa aga gtc cat ata cac cat gac 288
Gln Asn Ile Gly Ile Asp Lys Ile Lys Arg Val His Ile His His Asp
                85                  90                  95

cac gag cat cat gct gac cac gag cat cac tcg gac cat gag cat cac 336
His Glu His His Ala Asp His Glu His His Ser Asp His Glu His His
            100                 105                 110

tcg gac cac gag cat cac tcg gac cac gag cat cac tcg gac cac gag 384
Ser Asp His Glu His His Ser Asp His Glu His His Ser Asp His Glu
        115                 120                 125

cat cac tcg gac cac gag cac cat tcc cac cgc agt cac acg gtt gct 432
His His Ser Asp His Glu His His Ser His Arg Ser His Thr Val Ala
    130                 135                 140

ggt aaa aac aat cgg aaa gcc ttt tgt cca gac ctt gac tct gat aat 480
Gly Lys Asn Asn Arg Lys Ala Phe Cys Pro Asp Leu Asp Ser Asp Asn
145                 150                 155                 160

tca ggt aaa aat cct aga act agt cta ggg aaa gga tct cgc cca gca 528
Ser Gly Lys Asn Pro Arg Thr Ser Leu Gly Lys Gly Ser Arg Pro Ala
                165                 170                 175

gag cac atg aat ggt agg agg aac atc aag gag agt gca agc tct agt 576
Glu His Met Asn Gly Arg Arg Asn Ile Lys Glu Ser Ala Ser Ser Ser
            180                 185                 190

gaa gtg acc tcg gcg gta tac aac gct gtc tct gaa gga act cgc ttt 624
Glu Val Thr Ser Ala Val Tyr Asn Ala Val Ser Glu Gly Thr Arg Phe
        195                 200                 205

gta gag aca ata gag act cca aaa cct ggg aga cgc acc aaa gat gta 672
Val Glu Thr Ile Glu Thr Pro Lys Pro Gly Arg Arg Thr Lys Asp Val
    210                 215                 220

aac cct tct acc cca ccc agc atc acg gag aaa agc cga gtg ggc cgg 720
Asn Pro Ser Thr Pro Pro Ser Ile Thr Glu Lys Ser Arg Val Gly Arg
225                 230                 235                 240

ctg agt cgg cta gct agg aag aaa agc aat gag tct gtg agt gag ccc 768
Leu Ser Arg Leu Ala Arg Lys Lys Ser Asn Glu Ser Val Ser Glu Pro
                245                 250                 255

aga aag agc ttt atg tat tcc aga aac aca aat gac aat att cag gag 816
Arg Lys Ser Phe Met Tyr Ser Arg Asn Thr Asn Asp Asn Ile Gln Glu
            260                 265                 270

tgt ttc aat aca acc aag ctg ctg aca tcc cat ggc atg agc atc cag 864
Cys Phe Asn Thr Thr Lys Leu Leu Thr Ser His Gly Met Ser Ile Gln
        275                 280                 285

gct ctg ttg aat gca acg gaa ttt aac tat ctc tgc cca gcc atc atc 912
Ala Leu Leu Asn Ala Thr Glu Phe Asn Tyr Leu Cys Pro Ala Ile Ile
    290                 295                 300

aat caa att gat gct cgg gct tgt ctg att cat aca gca agt gag aag 960
Asn Gln Ile Asp Ala Arg Ala Cys Leu Ile His Thr Ala Ser Glu Lys
305                 310                 315                 320

aag gca gaa atc cct cca aag acc tat tct tta caa ata gcc tgg ctt 1008
Lys Ala Glu Ile Pro Pro Lys Thr Tyr Ser Leu Gln Ile Ala Trp Leu
                325                 330                 335

ggt ggc ttc ata gcc att tcc atc atc agt ttc ctg tct ctg ctg gga 1056
Gly Gly Phe Ile Ala Ile Ser Ile Ile Ser Phe Leu Ser Leu Leu Gly
            340                 345                 350

gtc atc ttg gtg cca ctc atg aac cgg gta ttt ttc aag ttc ctg ctg 1104
Val Ile Leu Val Pro Leu Met Asn Arg Val Phe Phe Lys Phe Leu Leu
        355                 360                 365

agc ttc ctc gtg gcg ctg gcc gtc gga acg ctg agt ggc gat gct ctg 1152
Ser Phe Leu Val Ala Leu Ala Val Gly Thr Leu Ser Gly Asp Ala Leu
    370                 375                 380

tta cat ctt ctc cca cac tct cat gca agt cat cag cac agt cat agc 1200
Leu His Leu Leu Pro His Ser His Ala Ser His Gln His Ser His Ser
385                 390                 395                 400

cat gaa gag cca gcg atg gaa atg aaa aga ggc ccc ctg ttc agc cac 1248
His Glu Glu Pro Ala Met Glu Met Lys Arg Gly Pro Leu Phe Ser His
                405                 410                 415

ctg tcg gct cag aat ata gaa gaa agc tcc tat ttt gat tcc acg tgg 1296
Leu Ser Ala Gln Asn Ile Glu Glu Ser Ser Tyr Phe Asp Ser Thr Trp
            420                 425                 430

aaa ggt ctg acg gct cta ggg ggc tta tat ttc atg ttt ctt gtg gaa 1344
Lys Gly Leu Thr Ala Leu Gly Gly Leu Tyr Phe Met Phe Leu Val Glu
        435                 440                 445

cac gta ctc aca ctg atc aag caa ttt aaa gat aag aaa aag aag aat 1392
His Val Leu Thr Leu Ile Lys Gln Phe Lys Asp Lys Lys Lys Lys Asn
    450                 455                 460

caa aag aaa cct gaa aat gat gag gat gtg gag agc aag aag cag ctg 1440
Gln Lys Lys Pro Glu Asn Asp Glu Asp Val Glu Ser Lys Lys Gln Leu
465                 470                 475                 480

tcc aaa tac gac tct cag ctt tcc tca aat gaa gag aag gtg gac cca 1488
Ser Lys Tyr Asp Ser Gln Leu Ser Ser Asn Glu Glu Lys Val Asp Pro
                485                 490                 495

ggg gaa cga cct gaa agc tat ctg cga gcc gac tcc caa gag ccc tcc 1536
Gly Glu Arg Pro Glu Ser Tyr Leu Arg Ala Asp Ser Gln Glu Pro Ser
            500                 505                 510

ccc ttt gat tcc cag cag ccg acg atg ttg gaa gag gaa gag gtc atg 1584
Pro Phe Asp Ser Gln Gln Pro Thr Met Leu Glu Glu Glu Glu Val Met
        515                 520                 525

ata gcc cat gca cac cca caa gaa gtc tac aat gaa tat gtg ccc agg 1632
Ile Ala His Ala His Pro Gln Glu Val Tyr Asn Glu Tyr Val Pro Arg
    530                 535                 540

ggc tgc aag aac aag tgc cat tca cac ttc cac gat acg ctg ggc cag 1680
Gly Cys Lys Asn Lys Cys His Ser His Phe His Asp Thr Leu Gly Gln
545                 550                 555                 560

tcc gac gac ctc atc cac cac cat cac gac tac cat cac att ctg cac 1728
Ser Asp Asp Leu Ile His His His His Asp Tyr His His Ile Leu His
                565                 570                 575

cac cac cac cac cag aac cac cac cct cac agc cac agc cag cgc tac 1776
His His His His Gln Asn His His Pro His Ser His Ser Gln Arg Tyr
            580                 585                 590

tct cga gag gag ctg aag gac gcc ggc att gcc aca ttg gcc tgg atg 1824
Ser Arg Glu Glu Leu Lys Asp Ala Gly Ile Ala Thr Leu Ala Trp Met
        595                 600                 605

gtg atc atg ggc gac ggg ctg cac aat ttc agt gac ggc ctt gct att 1872
Val Ile Met Gly Asp Gly Leu His Asn Phe Ser Asp Gly Leu Ala Ile
    610                 615                 620

ggt gct gcc ttc acc gag ggt ttg tcc agt ggg cta agc acc tct gtc 1920
Gly Ala Ala Phe Thr Glu Gly Leu Ser Ser Gly Leu Ser Thr Ser Val
625                 630                 635                 640

gct gtg ttc tgt cat gaa ctg cct cat gaa cta ggt gac ttt gct gtt 1968
Ala Val Phe Cys His Glu Leu Pro His Glu Leu Gly Asp Phe Ala Val
                645                 650                 655

ttg cta aag gct ggc atg act gtc aag cag gct gtg ctc tat aat gct     2016
Leu Leu Lys Ala Gly Met Thr Val Lys Gln Ala Val Leu Tyr Asn Ala
            660                 665                 670

ctg tca gcc atg ttg gcc tac ctt gga atg gca aca ggg ata ttc atc     2064
Leu Ser Ala Met Leu Ala Tyr Leu Gly Met Ala Thr Gly Ile Phe Ile
        675                 680                 685

ggg cat tat gca gaa aat gtt tct atg tgg ata ttc gca ctc act gcc     2112
Gly His Tyr Ala Glu Asn Val Ser Met Trp Ile Phe Ala Leu Thr Ala
    690                 695                 700

ggc ttg ttc atg tat gtc gct ctg gtt gac atg gtg cct gag atg ttg     2160
Gly Leu Phe Met Tyr Val Ala Leu Val Asp Met Val Pro Glu Met Leu
705                 710                 715                 720

cac aat gat gct agt gat cac gga tgc agc cgt tgg gga tat ttc ttc     2208
His Asn Asp Ala Ser Asp His Gly Cys Ser Arg Trp Gly Tyr Phe Phe
                725                 730                 735

ctg cag aat gct ggg ata ctt ctc ggt ttt gga att atg tta ctc att     2256
Leu Gln Asn Ala Gly Ile Leu Leu Gly Phe Gly Ile Met Leu Leu Ile
            740                 745                 750

tcc ata ttt gag cat aaa att gtg ttt cgt ata aat ttc taa             2298
Ser Ile Phe Glu His Lys Ile Val Phe Arg Ile Asn Phe
        755                 760                 765


<210>  6
<211>  765
<212>  PRT
<213>  Mus musculus

<400>  6

Met Ala Thr Asp Leu Ser Val Ile Met Ile Leu Thr Phe Ala Leu Trp
1               5                   10                  15

Val Thr Ser Pro Leu His Glu Leu Gln Ser Thr Ala Ala Phe Ser Gln
            20                  25                  30

Thr Thr Glu Lys Ile Asn Ser Asn Trp Glu Pro Gly Val Asn Val Asp
        35                  40                  45

Leu Ala Val Thr Met Gln Arg His His Leu Gln Gln Leu Phe Tyr Arg
    50                  55                  60

Tyr Gly Glu Asn Asp Ser Leu Ser Val Glu Gly Phe Arg Lys Leu Leu
65                  70                  75                  80

Gln Asn Ile Gly Ile Asp Lys Ile Lys Arg Val His Ile His His Asp
                85                  90                  95

His Glu His His Ala Asp His Glu His His Ser Asp His Glu His His
            100                 105                 110

Ser Asp His Glu His His Ser Asp His Glu His His Ser Asp His Glu
        115                 120                 125

His His Ser Asp His Glu His His Ser His Arg Ser His Thr Val Ala
    130                 135                 140

Gly Lys Asn Asn Arg Lys Ala Phe Cys Pro Asp Leu Asp Ser Asp Asn
145                 150                 155                 160

Ser Gly Lys Asn Pro Arg Thr Ser Leu Gly Lys Gly Ser Arg Pro Ala
                165                 170                 175

Glu His Met Asn Gly Arg Arg Asn Ile Lys Glu Ser Ala Ser Ser Ser
            180                 185                 190

Glu Val Thr Ser Ala Val Tyr Asn Ala Val Ser Glu Gly Thr Arg Phe
        195                 200                 205

Val Glu Thr Ile Glu Thr Pro Lys Pro Gly Arg Arg Thr Lys Asp Val
    210                 215                 220

Asn Pro Ser Thr Pro Pro Ser Ile Thr Glu Lys Ser Arg Val Gly Arg
225                 230                 235                 240

Leu Ser Arg Leu Ala Arg Lys Lys Ser Asn Glu Ser Val Ser Glu Pro
                245                 250                 255

Arg Lys Ser Phe Met Tyr Ser Arg Asn Thr Asn Asp Asn Ile Gln Glu
            260                 265                 270

Cys Phe Asn Thr Thr Lys Leu Leu Thr Ser His Gly Met Ser Ile Gln
        275                 280                 285

Ala Leu Leu Asn Ala Thr Glu Phe Asn Tyr Leu Cys Pro Ala Ile Ile
    290                 295                 300

Asn Gln Ile Asp Ala Arg Ala Cys Leu Ile His Thr Ala Ser Glu Lys
305                 310                 315                 320

Lys Ala Glu Ile Pro Pro Lys Thr Tyr Ser Leu Gln Ile Ala Trp Leu
                325                 330                 335

Gly Gly Phe Ile Ala Ile Ser Ile Ile Ser Phe Leu Ser Leu Leu Gly
            340                 345                 350

Val Ile Leu Val Pro Leu Met Asn Arg Val Phe Phe Lys Phe Leu Leu
        355                 360                 365

Ser Phe Leu Val Ala Leu Ala Val Gly Thr Leu Ser Gly Asp Ala Leu
    370                 375                 380

Leu His Leu Leu Pro His Ser His Ala Ser His Gln His Ser His Ser
385                 390                 395                 400

His Glu Glu Pro Ala Met Glu Met Lys Arg Gly Pro Leu Phe Ser His
                405                 410                 415

Leu Ser Ala Gln Asn Ile Glu Glu Ser Ser Tyr Phe Asp Ser Thr Trp
            420                 425                 430

Lys Gly Leu Thr Ala Leu Gly Gly Leu Tyr Phe Met Phe Leu Val Glu
        435                 440                 445

His Val Leu Thr Leu Ile Lys Gln Phe Lys Asp Lys Lys Lys Lys Asn
    450                 455                 460

Gln Lys Lys Pro Glu Asn Asp Glu Asp Val Glu Ser Lys Lys Gln Leu
465                 470                 475                 480

Ser Lys Tyr Asp Ser Gln Leu Ser Ser Asn Glu Glu Lys Val Asp Pro
                485                 490                 495

Gly Glu Arg Pro Glu Ser Tyr Leu Arg Ala Asp Ser Gln Glu Pro Ser
            500                 505                 510

Pro Phe Asp Ser Gln Gln Pro Thr Met Leu Glu Glu Glu Glu Val Met
        515                 520                 525

Ile Ala His Ala His Pro Gln Glu Val Tyr Asn Glu Tyr Val Pro Arg
    530                 535                 540

Gly Cys Lys Asn Lys Cys His Ser His Phe His Asp Thr Leu Gly Gln
545                 550                 555                 560

Ser Asp Asp Leu Ile His His His His Asp Tyr His His Ile Leu His
                565                 570                 575

His His His His Gln Asn His His Pro His Ser His Ser Gln Arg Tyr
            580                 585                 590

Ser Arg Glu Glu Leu Lys Asp Ala Gly Ile Ala Thr Leu Ala Trp Met
        595                 600                 605

Val Ile Met Gly Asp Gly Leu His Asn Phe Ser Asp Gly Leu Ala Ile
    610                 615                 620

Gly Ala Ala Phe Thr Glu Gly Leu Ser Ser Gly Leu Ser Thr Ser Val
625                 630                 635                 640

Ala Val Phe Cys His Glu Leu Pro His Glu Leu Gly Asp Phe Ala Val
                645                 650                 655

Leu Leu Lys Ala Gly Met Thr Val Lys Gln Ala Val Leu Tyr Asn Ala
            660                 665                 670

Leu Ser Ala Met Leu Ala Tyr Leu Gly Met Ala Thr Gly Ile Phe Ile
        675                 680                 685

Gly His Tyr Ala Glu Asn Val Ser Met Trp Ile Phe Ala Leu Thr Ala
    690                 695                 700

Gly Leu Phe Met Tyr Val Ala Leu Val Asp Met Val Pro Glu Met Leu
705                 710                 715                 720

His Asn Asp Ala Ser Asp His Gly Cys Ser Arg Trp Gly Tyr Phe Phe
                725                 730                 735

Leu Gln Asn Ala Gly Ile Leu Leu Gly Phe Gly Ile Met Leu Leu Ile
            740                 745                 750

Ser Ile Phe Glu His Lys Ile Val Phe Arg Ile Asn Phe
        755                 760                 765

<210> 7
<211> 25
<212> DNA
<213> Artificial

<220>
<223> antisense

<400> 7
cggaaacagc gcgagtgtct tttgt 25


<210> 8
<211> 25
<212> DNA
<213> Artificial

<220>
<223> antisense

<400> 8
accgtgtgca aagaaacgtc atcat 25

## Description

### Technical Field

The present invention relates to an agent capable of controlling the function of an antigen-presenting cell. More specifically, the present invention relates to an agent capable of controlling an immune response reaction mediated by activation of an antigen-presenting cell.

### Background Art

One of the systems for maintaining human health is the immune system. Controlling immune responses is of paramount importance from the viewpoint of development of a vaccine aiming at protection against infections, treatment of autoimmune diseases, conquest of cancer and the like, and suppression of graft rejection in organ transplant. To date, for the purpose of controlling the immune responses, various immunopotentiators, for example, microbial components such as BCG, have been used and immunosuppressants derived from microbial components and synthetic steroids have been administered to the human body. However, these microbial components and synthetic steroids involve various adverse reactions, and are potentially harmful to the human body. Also, some problems with the use thereof as drugs, including attenuation of the effects thereof during long-term use, have been pointed out to date.

Antigen-presenting cells such as dendritic cells have a central role in the control of the intensity and quality of immune responses, and are applied to immunotherapies for autoimmune diseases, allergic diseases, and cancer, and the like. Research is also ongoing into culture methods for controlling the growth or activation of antigen-presenting cells, and the like, but still there is a demand for a method of more efficiently and specifically controlling the function of an antigen-presenting cell.

The prior art is described in detail in Guermonprez P, Valladeau J, Zitvogel L, Thery C, Amigorena S. Antigen presentation and T cell stimulation by dendritic cells. Annu Rev. Immunol. 20, 621-627 (2002), Banchereau J, Briere F, Caux C, Davoust J, Lebecque S, Liu YJ, Pulendran B, Palucka K. Immunobiology of dendritic cells. Annu Rev. Immunol. 18, 761-781 (2000), and Steinman RM, Mellman I. Immunotherapy: bewitched, bothered, and bewildered no more. Science 305, 197-200 (2004).

### Disclosure of the Invention

It is an object of the present invention to provide a novel agent for controlling the function of an antigen-presenting cell, specifically to provide an agent capable of controlling immune responses mediated by activation of an antigen-presenting cell (controlling as mentioned herein includes both enhancement and suppression depending on the object). It is another object of the present invention to apply the agent to various diseases, including treatment of autoimmune diseases, treatment of allergic diseases, suppression of graft rejection in organ transplant, and the like, and still other objects are to develop a method of enhancing immune responses to cancer, and to efficiently develop a vaccine.

The present inventors diligently investigated in view of the above-described objects, and found that by controlling the zinc ion level, the function of an antigen-presenting cell such as a dendritic cell can be controlled. Furthermore, the present inventors confirmed that the function control of an antigen-presenting cell by controlling the zinc ion concentration was reversible, and developed the present invention. Accordingly, the present invention provides:
[1] An agent for controlling the function of an antigen-presenting cell, comprising a substance capable of controlling the intracellular zinc ion concentration as the active ingredient.
[2] The agent described in [1] above, wherein the substance capable of controlling the zinc ion concentration is a zinc ion.
[3] The agent described in [2] above, wherein the zinc ion is introduced into a cell through a zinc ionophore.
[4] The agent described in [1] above, wherein the substance capable of controlling the zinc ion concentration is a zinc ion chelator.
[5] The agent described in [4] above, wherein the zinc ion chelator is at least one selected from the group consisting of 2,3-dimercapto-1-propanesulfonic acid (DMPS), N,N,N',N'-tetrakis(2-pyridylmethyl)ethylenediamine (TPEN), ethylenediamine (EDTA) and N-(6-methoxy-8-quinolyl)-p-toluenesulfonamide (TSQ).
[6] The agent described in [1] above, wherein the substance capable of controlling the zinc ion concentration is a substance regulating the expression and/or function of a zinc ion-requiring protein.
[7] The agent described in [6] above, wherein the zinc ion-requiring protein is at least one selected from the group consisting of Raf-1, PKCα, GEF-H1, HDAC, SQSTM1, ubiquitin-protein ligase E3, ubiquitin-conjugating enzyme E2, metallothionein, phosphatase, Snail, TRAF6, Paxillin, Zyxin and Jade-1.
[8] The agent described in [1] above, wherein the substance capable of controlling the zinc ion concentration is a substance regulating the expression and/or function of a zinc ion transporter.
[9] The agent described in [8] above, wherein the zinc ion transporter is at least one selected from the group consisting of human ZIPs including LIV family and human CDFs.
[10] The agent described in any of [1] to [9] above, wherein the antigen-presenting cell is at least one selected from the group consisting of a dendritic cell, a macrophage, a Langerhans' cell, a B cell, a thymic epithelial cell, a synovial cell, a vascular endothelial cell and a keratinocyte.
[11] The agent described in [10] above, wherein the antigen-presenting cell is a dendritic cell.
[12] A prophylactic and/or therapeutic drug for a disease mediated by the immune system, comprising the function controlling agent described in any of [1] to [11] above.
[13] The prophylactic and/or therapeutic drug described in [12] above, wherein the disease mediated by the immune system is at least one selected from the group consisting of autoimmune disease, allergic disease, graft rejection in organ transplant, an immune response reduction due to infection or tumorigenesis, and an abnormal immune response during vaccination.
[14] A prophylactic and/or therapeutic drug for a disease mediated by the immune system, comprising a substance capable of controlling the intracellular zinc ion concentration as the active ingredient.
[15] The prophylactic and/or therapeutic drug described in [14] above, wherein the substance capable of controlling the zinc ion concentration is a zinc ion.
[16] The prophylactic and/or therapeutic drug described in [15] above, wherein the zinc ion is introduced into a cell through a zinc ionophore.
[17] The prophylactic and/or therapeutic drug described in [14] above, wherein the substance capable of controlling the zinc ion concentration is a zinc ion chelator.
[18] The prophylactic and/or therapeutic drug described in [17] above, wherein the zinc ion chelator is at least one selected from the group consisting of 2,3-dimercapto-1-propanesulfonic acid (DMPS), N,N,N',N'-tetrakis(2-pyridylmethyl)ethylenediamine (TPEN), ethylenediamine (EDTA) and N-(6-methoxy-8-quinolyl)-p-toluenesulfonamide (TSQ).
[19] The prophylactic and/or therapeutic drug described in [14] above, wherein the substance capable of controlling the zinc ion concentration is a substance regulating the expression and/or function of a zinc ion-requiring protein.
[20] The prophylactic and/or therapeutic drug described in [19] above, wherein the zinc ion-requiring protein is at least one selected from the group consisting of Raf-1, PKCα, GEF-H1, HDAC, SQSTM1, ubiquitin-protein ligase E3, ubiquitin-conjugating enzyme E2, metallothionein, phosphatase, Snail, TRAF6, Paxillin, Zyxin and Jade-1.
[21] The prophylactic and/or therapeutic drug described in [14] above, wherein the substance capable of controlling the zinc ion concentration is a substance regulating the expression and/or function of a zinc ion transporter.
[22] The prophylactic and/or therapeutic drug described in [21] above, wherein the zinc ion transporter is at least one selected from the group consisting of human ZIPs including LIV family and human CDFs.
[23] The prophylactic and/or therapeutic drug described in any of [14] to [22] above, wherein the disease mediated by the immune system is at least one selected from the group consisting of autoimmune disease, allergic disease, graft rejection in organ transplant, an immune response reduction due to infection or tumorigenesis and an abnormal immune response during vaccination.
[24] The prophylactic and/or therapeutic drug described in any of [14] to [23] above, wherein the antigen-presenting cell is at least one selected from the group consisting of a dendritic cell, a macrophage, a Langerhans' cell, a B cell, a thymic epithelial cell, a synovial cell, a vascular endothelial cell and a keratinocyte.
[25] The prophylactic and/or therapeutic drug described in [24] above, wherein the antigen-presenting cell is a dendritic cell.
[26] A method of controlling the function of an antigen-presenting cell, comprising controlling the intracellular zinc ion concentration in vitro.
[27] The method described in [26] above, wherein the zinc ion concentration is controlled by introducing a zinc ion into a cell.
[28] The method described in [27] above, wherein the zinc ion is introduced into the cell through a zinc ionophore.
[29] The method described in [26] above, wherein the zinc ion concentration is controlled by a zinc ion chelator.
[30] The method described in [29] above, wherein the zinc ion chelator is at least one selected from the group consisting of 2,3-dimercapto-1-propanesulfonic acid (DMPS), N,N,N',N'-tetrakis(2-pyridylmethyl)ethylenediamine (TPEN), ethylenediamine (EDTA) and N-(6-methoxy-8-quinolyl)-p-toluenesulfonamide (TSQ).
[31] The method described in [26] above, wherein the zinc ion concentration is controlled by regulating the expression and/or function of a zinc ion-requiring protein.
[32] The method described in [31] above, wherein the zinc ion-requiring protein is at least one selected from the group consisting of Raf-1, PKCα, GEF-H1, HDAC, SQSTM1, ubiquitin-protein ligase E3, ubiquitin-conjugating enzyme E2, metallothionein, phosphatase, Snail, TRAF6, Paxillin, Zyxin and Jade-1.
[33] The method described in [26] above, wherein the zinc ion concentration is controlled by regulating the expression and/or function of a zinc ion transporter.
[34] The method described in [33] above, wherein the zinc ion transporter is at least one selected from the group consisting of human ZIPs including LIV family and human CDFs.
[35] The method described in any of [26] to [34] above, wherein the antigen-presenting cell is at least one selected from the group consisting of a dendritic cell, a macrophage, a Langerhans' cell, a B cell, a thymic epithelial cell, a synovial cell, a vascular endothelial cell and a keratinocyte.
[36] A screening method for a compound capable of controlling the function of an antigen-presenting cell, comprising measuring the intracellular zinc ion concentration.
[37] The method described in [36] above, wherein the antigen-presenting cell is at least one selected from the group consisting of a dendritic cell, a macrophage, a Langerhans' cell, a B cell, a thymic epithelial cell, a synovial cell, a vascular endothelial cell and a keratinocyte.
[38] The method described in [37] above, wherein the antigen-presenting cell is a dendritic cell.
[39] A use of a substance capable of controlling the intracellular zinc ion concentration, for producing a function controlling agent for an antigen-presenting cell.
[40] A use of the function controlling agent described in any of [1] to [11] above, for producing a prophylactic and/or therapeutic drug for a disease mediated by the immune system.
[41] A use of a substance capable of controlling the intracellular zinc ion concentration, for producing a prophylactic and/or therapeutic drug for a disease mediated by the immune system.

### Brief Description of the Drawings

Figure 1 is a drawing showing that when dendritic cells are treated with a zinc ion chelator, the expression of MHC class I, class II, and CD86 molecules on the cell surface thereof is enhanced.
Figure 2 is a drawing showing that when dendritic cells are treated with a zinc ion and a zinc ionophore, LPS-stimulated enhancement of expression of MHC II and CD86 molecules and IL-12 production in the dendritic cells are suppressed.

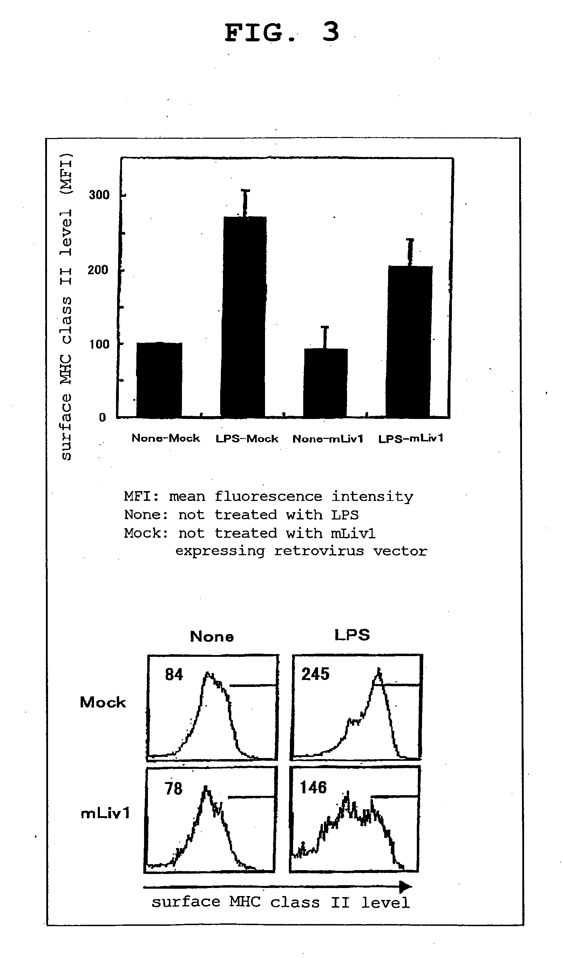

Figure 3 is a drawing showing that on dendritic cells allowed to express mLiv1 using a retrovirus vector, LPS-stimulated enhancement of expression of MHC class II molecule is suppressed.

### Best Mode for Embodying the Invention

In the present invention, by "controlling a zinc ion concentration" is not only literally meant making the intracellular zinc ion concentration (amount) increase or decrease, but also meant an effect which can ultimately induce the same phenomena as those caused by an increase or decrease of the intracellular zinc ion concentration, and in such a case, the wording is not limited by the level of the intracellular zinc ion concentration. In the present invention, a cell to be a target of control of the zinc ion concentration is an antigen-presenting cell such as a dendritic cell, a macrophage, a Langerhans' cell, a B cell, or a thymic epithelial cell, or a cell expected to acquire antigen-presenting capability, such as a synovial cell, a vascular endothelial cell, or a keratinocyte. The cell is preferably a dendritic cell. Herein, a cell to be a target of control is sometimes referred to as "an antigen-presenting cell" for the sake of convenience.

A "substance capable of controlling an intracellular zinc ion concentration" includes a "zinc ion", a "zinc ion chelator", a "zinc ionophore", a "substance regulating the expression and/or function of a zinc ion-requiring protein", a "substance regulating the expression and/or function of a zinc ion transporter" and the like. The "zinc ion" and "zinc ionophore" can directly increase the intracellular zinc ion concentration of the antigen-presenting cell by addition thereof, whereas the "zinc ion chelator" is capable of directly reducing the zinc ion concentration in an antigen-presenting cell by being added, whereby the function of an antigen-presenting cell can be controlled. The "substance regulating the expression and/or function of a zinc ion-requiring protein" and "a substance regulating the expression and/or function of a zinc ion transporter" are capable of altering the action of the zinc ion on an antigen-presenting cell and controlling the function of the antigen-presenting cell by regulating the expression and/or function of the zinc ion-requiring protein, or by regulating the expression and/or function of the zinc ion transporter. When the "substance regulating the expression and/or function of a zinc ion-requiring protein" or the "substance regulating the expression and/or function of a zinc ion transporter" is a substance regulating in the direction of promoting the expression or function, the responsiveness of the cell to zinc ions can be more enhanced, and when the substance is a substance regulating in the direction of suppressing the expression or function, the responsiveness of the cell to zinc ions can be suppressed more. In the present invention, "controlling" the zinc ion concentration means both positive and negative control.

An antigen-presenting cell presents 1) an exogenous substance (foreign substance) derived from infection and the like and 2) an endogenous protein resulting from tumorigenesis of one's own body or a cell, viral infection and the like to a cell having TCR (for example, CD4⁺T cell, CD8⁺T cell, DNT cell, NKT cell and the like). Therein, protection against infections occurs and cells that are undesirable for one's own body are removed, on the basis of distinguishing between self and non-self. In the present invention, by "controlling" these functions of an antigen-presenting cell, an immune response preferable for the maintenance of life is induced. "Controlling" the function of an antigen-presenting cell more specifically means defining the quality and quantity of an immune response after antigen presentation. As used herein, "quality" means control of responses of Th1 and Th2 cells, immunotolerance and the like in CD4⁺T cells, or control of responses of CTL, which has antigen-specific cytotoxic activity, immunotolerance and the like in CD8⁺T cells; "quantity" literally means the intensity of the immune response. Also, "controlling" the function of an antigen-presenting cell means the activation or inactivation of the antigen-presenting cell per se. Specifically, "controlling" the function of an antigen-presenting cell means function controlling of one or more items selected from among 1) control of expression of MHC class I or II molecule, 2) control of expression of a co-stimulatory molecule such as CD80, CD86, or CD40, 3) control of expression of a cytokine such as IL-1, IL-6, IL-10, IL-12, IFN-α/β, TNF-α, or TGF-β, 4) control of uptake of an exogenous or endogenous antigen, 5) control of migration capability of an antigen-presenting cell, and the like.

The "zinc ion" is introduced into the antigen-presenting cell through a zinc ionophore. Namely, the zinc ion is introduced in the form of a complex with the zinc ionophore into the antigen-presenting cell. The zinc ionophore includes a variety of compounds commonly used in the art, preferably those commercially available. It is exemplified by zinc pyrithione, heterocyclic amine, dithiocarbamate, vitamins and the like.

The "zinc ion chelator" is a substance that can form a complex with the zinc ion in the antigen-presenting cell, thereby removing the zinc ion from the cell, and includes, for example, 2,3-dimercapto-1-propanesulfonic acid (DMPS), N,N,N',N'-tetrakis(2-pyridylmethyl)ethylenediamine (TPEN), ethylenediamine (EDTA), N-(6-methoxy-8-quinolyl)-p-toluenesulfonamide (TSQ) and the like. All of them are commercially available. The amount of the zinc ion chelator as a substance capable of controlling the concentration of the zinc ion to be comprised as an active ingredient in the function controlling agent of the present invention for an antigen-presenting cell or the prophylactic and/or therapeutic drug of the present invention for a disease mediated by the immune system (hereinafter, sometimes simply referred to as "prophylactic and/or therapeutic drug") is appropriately determined within a range in which the control of the intracellular zinc ion concentration is possible, in each agent or drug. One or more types of zinc ion chelators may be comprised therein. When two or more types of zinc ion chelators are comprised, their amounts are appropriately set depending on their types.

The "substance regulating the expression and/or function of a zinc ion-requiring protein" is not particularly limited by its mechanism of action, as long as it can eventually regulate the expression and/or function of a zinc ion-requiring protein, and the regulation may be at the gene level or protein level. The regulation at the gene level includes transcriptional regulation, gene expression regulation and the like. The regulation at the protein level includes metabolic regulation, phosphorylation, dephosphorylation, glycosylation, lipidation, coordinate bond with zinc, degradation, ubiquitination, acetylation and the like. The "zinc ion-requiring protein" is a protein that requires zinc for its functional expression in the antigen-presenting cell, and includes, for example, a protein having a zinc finger, a protein having a RING finger, a protein having an LIM domain, a protein having a PHD zinc finger and the like. The zinc finger is a motif having a unique nucleic acid-binding ability, which can form a higher-order structure only by coordination of amino acids such as cysteine and histidine to the zinc. For example, a protein having a zinc finger is exemplified by a transcription factor Snail which is a master regulator in a phenomenon called epithelial-mesenchymal transition (phenomenon in which cells usually tightly adhering to each other acquire mobility by releasing the adhesion to their adjacent cells and migrate to other sites during morphogenesis in the early development of humans and other organisms and during wound healing and cancer metastasis and the like), PKCα, GEF-H1, HDAC, SQSTM1 and the like. The RING finger may be said to be a variation of the zinc finger and it has been suggested that it is involved in a protein-protein interaction. The reported protein having the RING finger includes those having ubiquitin-protein ligase E3 activity, those exhibiting ubiquitin-conjugating enzyme E2 binding, and those exhibiting TRAF6 (a signaling molecule of TNFα) binding. The LIM domain consists of 60 amino acids with the positions of 6 cysteines and 1 histidine being conserved. Although this domain is structurally very similar to the zinc finger, its DNA binding ability has not been known. It also functions as a domain mediating the binding to PKC. A protein having the LIM domain is exemplified by Paxillin, Zyxin and the like, which are involved in cytoskeletal control. The PHD zinc finger is a zinc finger-like domain in nuclear proteins suggested to be involved in chromatin-mediated transcriptional control and is expected to have the DNA binding ability. A protein having a PHD zinc finger is exemplified by Jade-1 which is involved in histone acetylation and the like.

The "substance regulating the expression and/or function of a zinc ion-requiring protein" may be a known compound or a novel compound to be developed in the future. It may be a low-molecular-weight compound or a high-molecular-weight compound. In this context, the low-molecular-weight compound is a compound having a molecular weight of less than approximately 3000, which includes, for example, an organic compound and a derivative thereof and an inorganic compound usually available as a pharmaceutical agent, and refers to a compound and a derivative thereof produced by making full use of organic synthesis or the like, a naturally occurring compound and a derivative thereof, small nucleic acid molecules such as promoter, a variety of metals and the like, and desirably an organic compound and a derivative thereof and a nucleic acid molecule available as a pharmaceutical agent. The high-molecular-weight compound is a compound having a molecular weight of not lower than approximately 3000, which includes, for example, protein, polynucleic acid, polysaccharide and combinations thereof, and is desirably a protein. These low-molecular-weight or high-molecular-weight compounds, if they are known, are commercially available or can be obtained by way of steps such as collection, production and purification according to the respective reported documents. These compounds may be naturally occurring or prepared by genetic engineering, or can also be obtained by semi-synthesis and the like. Specifically, the expression of a zinc ion-requiring protein Snail is regulated by MAPK through TGF-β or FGF (Peinado, H., Quintanilla, M. & Cano, A. Transforming growth factor beta-1 induces snail transcription factor in epithelial cell lines: mechanisms for epithelial mesenchymal transitions. J. Biol. Chem. 278, 21113-23 (2003)). Moreover, Snail is activated by LIV1 (as described below), a zinc ion transporter (Yamashita, S., Miyagi, C., Fukada, T., Kagara, N., Che, Y.-S. & Hirano, T. Zinc transporter LIVI controls epithelial-mesenchymal transition in zebrafish gastrula organizer. Nature 429, 298-302 (2004)). Details for Raf1, GEF-H1, PKCα, phosphatase and metallothionein exemplified as other zinc ion-requiring proteins are respectively described in Jirakulaporn T, Muslin AJ. Cation diffusion facilitator proteins modulate Raf-1 activity. J. Biol. Chem. 279, 27807-15 (2004), Krendel M, Zenke FT, Bokoch GM. Nucleotide exchange factor GEF-H1 mediates cross-talk between microtubules and the actin cytoskeleton. Nature Cell Biology 4, 294-301 (2002), Korichneva I, Hoyos B, Chua R, Levi E, Hammerling U. Zinc release from protein kinase C as the common event during activation by lipid second messenger or reactive oxygen. J. Biol. Chem. 277, 44327-31 (2002), Haase H, Maret W. Intracellular zinc fluctuations modulate protein tyrosine phosphatase activity in insulin/insulin-like growth factor-1 signaling. Exp. Cell Research 291, 289-298 (2003), and Seikagaku Jiten (3rd ed.), Tokyo Kagaku Dozin Co., Ltd., pp.1393 (1998). Details for TRAF6, Paxillin, Zyxin and Jade-1 are respectively described in Kobayashi N, Kadono Y, Naito A, Matsumoto K, Yamamoto T, Tanaka S, Inoue J. Segregation of TRAF6-mediated signaling pathways clarifies its role in osteoclastogenesis. The EMBO J. 20, 1271-1280 (2001), Brown MC, Perrotta JA, Turner CE. Identification of LIM3 as the principal determinant of paxillin focal adhesion localization and characterization of a novel motif on paxillin directing vinculin and focal adhesion kinase binding. J. Cell Biol. 135, 1109-1123 (1996), Sadler I, Crawford AW, Michelsen JW, Beckerle MC. Zyxin and cCRP: two interactive LIM domain proteins associated with the cytoskeleton. J. Cell Biol. 119, 1573-1587 (1992), and Panchenko MV, Zhou MI, Cohen HT. von Hippel-Lindau partner Jade-1 is a transcriptional co-activator associated with histone acetyltransferase activity. J. Biol. Chem. 279, 56032-56041 (2004). Furthermore, details for SQSTM1 and HDAC are respectively described in Joung I, Strominger JL, Shin J. Molecular cloning of a phosphotyrosine-independent ligand of the p561ck SH2 domain. Proc Natl Acad Sci USA, 93, 5991-5995 (1996) and Kawaguchi Y, Kovacs JJ, McLaurin A, Vance JM, Ito A, Yao TP. The deacetylase HDAC6 regulates aggresome formation and cell viability in response to misfolded protein stress. Cell 115, 727-738 (2003).

The "substance regulating the expression and/or function of a zinc ion transporter" is not particularly limited by its mechanism of action, as long as it can eventually regulate the expression and/or function of a zinc ion transporter, and the regulation may be at the gene level or protein level. The regulation at the gene level includes transcriptional regulation, gene expression regulation and the like. The regulation at the protein level includes metabolic regulation, phosphorylation, dephosphorylation, glycosylation, lipidation, coordinate bond with zinc, degradation, ubiquitination, acetylation and the like. The "zinc ion transporter" includes, for example, human ZIPs including LIV family (e.g., BAB70848, hZip4, BIGM103, KIAA0062, KIAA1265, hLiv-1, AAH08853, hKE4, XP_208649, hZIP1, hZIP2, hZIP3, BAA92100, BAC04504), human CDFs (e.g., hZnt-5, hZnt-7, hZnt-1, hZnt-6, hZnt-3, hZnt-2, hZnt-8, hZnt-4, hZnt-9) and the like. LIV1 was originally identified as an estrogen-controlled breast cancer protein and recently has been revealed to belong to a ZIP zinc ion transporter subfamily (Zrt⁻, Irt-like protein) called LZT (LIV-1 subfamily of ZIP zinc ion transporter) (Taylor, K. M. & Nicholson, R. I. The LZT proteins; the LIV-1 subfamily of zinc transporters. Biochim. Biophys. Acta 1611, 16-30 (2003)), and to function as a zinc ion transporter (Taylor, K. M., Morgan, H. E., Johnson, A., Hadley, L. J. & Nicholson, R. I. Structure-function analysis of LIV-1, the breast cancer-associated protein that belongs to a new subfamily of zinc transporters. Biochem. J. 375, 51-9 (2003)). In addition, CDF (cation diffusion facilitator) and the like are included. For example, the expression of the zinc ion transporter LIV1 is regulated by STAT3 (Yamashita, S., Miyagi, C., Fukada, T., Kagara, N., Che, Y.-S. & Hirano, T. Zinc transporter LIVI controls epithelial-mesenchymal transition in zebrafish gastrula organizer. Nature 429, 298-302 (2004)).

The "substance regulating the expression and/or function of a zinc ion transporter" may be a known compound or a novel compound to be developed in the future. It may be a low-molecular-weight compound or a high-molecular-weight compound. In this context, the low-molecular-weight compound is a compound having a molecular weight of less than approximately 3000, which includes, for example, an organic compound and a derivative thereof and an inorganic compound usually available as a pharmaceutical agent, and refers to a compound and a derivative thereof produced by making full use of organic synthesis or the like, a naturally occurring compound and a derivative thereof, small nucleic acid molecules such as promoter, a variety of metals and the like, and desirably an organic compound and a derivative thereof and a nucleic acid molecule available as a pharmaceutical agent. The high-molecular-weight compound is a compound having a molecular weight of not lower than approximately 3000, which includes, for example, protein, polynucleic acid, polysaccharide and combinations thereof, and is desirably a protein. These low-molecular-weight or high-molecular-weight compounds, if they are known, are commercially available or can be obtained by way of steps such as collection, production and purification according to the respective reported documents. These compounds may be naturally occurring or prepared by genetic engineering, or can also be obtained by semi-synthesis and the like.

Since the zinc ion transporter LIV1 enhances the activity (particularly, repressor activity) of the zinc ion-requiring protein Snail (Yamashita, S., Miyagi, C., Fukada, T., Kagara, N., Che, Y.-S. & Hirano, T. Zinc transporter LIVI controls epithelial-mesenchymal transition in zebrafish gastrula organizer. Nature 429, 298-302 (2004)), an example of the "substance regulating the expression and/or function of a zinc ion-requiring protein" in the present invention includes the following:
(1) DNA according to any of the following a) to d):
   a) DNA encoding a protein (LIV1 protein) having an amino acid sequence described in SEQ ID NO: 2, 4, or 6;
   b) DNA (LIV1 gene) consisting of a sequence described in SEQ ID NO: 1, 3, or 5;
   c) DNA encoding a protein (protein analogous to LIV1) having an amino acid sequence described in SEQ ID NO: 2, 4, or 6 with substitution, deletion, insertion and/or addition of one or more amino acid sequences therein;
   d) DNA hybridizing to a sequence described in SEQ ID NO: 1, 3, or 5 under stringent condition;
(2) a vector in which DNA according to any of the following a) to d) is inserted:
   a) DNA encoding a protein having an amino acid sequence described in SEQ ID NO: 2, 4, or 6;
   b) DNA consisting of a sequence described in SEQ ID NO: 1, 3, or 5;
   c) DNA encoding a protein having an amino acid sequence described in SEQ ID NO: 2, 4, or 6 with substitution, deletion, insertion and/or addition of one or more amino acid sequences therein;
   d) DNA hybridizing to a sequence described in SEQ ID NO: 1, 3, or 5 under stringent condition;
(3) a protein encoded by DNA according to any of the following a) to d):
   a) DNA encoding a protein having an amino acid sequence described in SEQ ID NO: 2, 4, or 6;
   b) DNA consisting of a sequence described in SEQ ID NO: 1, 3, or 5;
   c) DNA encoding a protein having an amino acid sequence described in SEQ ID NO: 2, 4, or 6 with substitution, deletion, insertion and/or addition of one or more amino acid sequences therein;
   d) DNA hybridizing to a sequence described in SEQ ID NO: 1, 3, or 5 under stringent condition;
(4) An antisense oligonucleotide whose target sequence is DNA consisting of a sequence described in SEQ ID NO: 1, 3, or 5;
(5) double-stranded RNA having a sequence identical or similar to a portion of DNA consisting of a sequence described in SEQ ID NO: 1, 3, or 5;
(6) an antibody against a protein having an amino acid sequence described in SEQ ID NO: 2, 4, or 6;
(7) a substance (including natural and non-natural products) that associates with a protein having an amino acid sequence described in SEQ ID NO: 2, 4, or 6 and regulates a function of the protein.

The substances (1) to (3) positively regulate the expression and/or function of the zinc ion-requiring protein Snail (enhancing the expression and/or activating the function), and the substances (4) and (5) negatively regulate the expression and/or function of Snail (reducing the expression and/or suppressing the function).

The LIV1 protein can be prepared by a variety of methods well known to those skilled in the art. For example, the protein can be produced by a transformant and obtained by purification thereof, wherein the transformant bears a vector in which DNA (LIV1 gene) consisting of a base sequence described in SEQ ID NO: 1, 3, or 5 is inserted. The vector to be used can be appropriately selected depending on translation systems used for the protein production. Moreover, LIV1 is known to be expressed in hormonal tissues such as the breast, prostate, pituitary gland and brain (Taylor, K. M. & Nicholson, R. I. The LZT proteins; the LIV-1 subfamily of zinc transporters. Biochim. Biophys. Acta 1611, 16-30 (2003)). The LIV1 protein can be purified from LIV1-expressing cell extracts by preparing anti-LIV1 protein antibodies by a well known method and producing an affinity column with the antibodies.

A protein analogous to LIV1 is a protein capable of regulating the activity of a zinc ion-requiring protein (e.g., Snail) and can include, for example, a protein consisting of an amino acid sequence described in SEQ ID NO: 2, 4, or 6 with substitution, deletion, insertion and/or addition of one or more amino acid sequences therein, and a protein encoded by DNA hybridizing to DNA consisting of a base sequence described in SEQ ID NO: 1, 3, or 5 under stringent condition.

The protein analogous to LIV1 can be prepared for example, by a method involving performing hybridization utilizing a base sequence encoding LIV1, producing a transformant with the obtained DNA with high homology and allowing the transformant to produce the desired protein. An example of a method for obtaining the DNA with high homology can include a method involving performing hybridization under stringent hybridization condition using a portion of the base sequence described in SEQ ID NO: 1, 3, or 5 as a probe to obtain such a DNA from cells or the like of human or non-human vertebrate.

Those skilled in the art can appropriately select the above stringent hybridization condition. By way of example, prehybridization is performed overnight at 42°C in a hybridization solution containing 25% formamide or 50% formamide for more stringent condition, 4 x SSC, 50 mM Hepes pH 7.0, 10 x Denhardt's solution, and 20 µg/mL denatured salmon sperm DNA, and then hybridization is performed by adding a labeled probe and incubating overnight at 42°C. Subsequent washing can be performed under condition of a washing liquid and temperature of approximately "1 x SSC, 0.1% SDS, at 37°C", approximately "0.5 x SSC, 0.1% SDS, at 42°C" for more stringent condition, and approximately "0.2 x SSC, 0.1% SDS, at 65°C" for even more stringent condition. Thus, it can be expected that the more stringent the washing condition of the hybridization is, the higher the homology of the isolated DNA to the probe sequence is. However, the combinations of the SSC, SDS and temperature conditions are described for purposes of only exemplification, and those skilled in the art can appropriately combine the above-described or other determinants of the stringency of hybridization (e.g., probe concentration, probe length, hybridization reaction period and the like), thereby achieving a stringency similar to those described above.

A polypeptide encoded by the DNA isolated using such a hybridization technique generally has high homology to LIV1 in the amino acid sequence. The high homology refers to at least 40% or higher, preferably 60% or higher, more preferably 80% or higher, even more preferably 90% or higher, even still more preferably 95% or higher, particularly preferably 97% or higher (e.g., 98 to 99%) of sequence homology. An amino acid sequence identity can be determined by, for example, algorithm BLAST (Karlin and Altschul, Proc. Natl. Acad. Sci. USA 87: 2264-2268, 1990; and Proc. Natl. Acad. Sci. USA 90: 5873-5877, 1993). Based on this algorithm, a program called BLASTX has been developed (Altschul et al., J. Mol. Biol. 215: 403-410, 1990). When amino acid sequences are analyzed with the use of BLASTX, parameters are set to, for example, score=50 and word length=3. When BLAST and Gapped BLAST programs are used, the respective default parameters of the programs are used. The specific ways of these analysis methods are known (http://www.ncbi.nlm.nih.gov.)

The protein analogous to LIV1 may also be prepared by another known means. For example, LIV1 DNA is artificially modified by deletion mutant production using exonuclease and site-directed mutagenesis such as cassette mutagenesis, and the modified LIV1 DNA can be used to prepare the desired protein.

Another preferable example of the substance available as the "substance regulating the expression and/or function of a zinc ion-requiring protein" can include DNA consisting of the sequence described in SEQ ID NO: 1, 3, or 5. The above described DNA encodes LIV1 protein, and when introduced into cells, serves as a substance capable of indirectly regulating the activity of a zinc ion-requiring protein such as Snail, through LIV1 protein expression.

The above described DNA can also be prepared from a cDNA of a LIV1-expressing cell by hybridization techniques well known to those skilled in the art using a portion of the sequence described in SEQ ID NO: 1, 3, or 5 as a probe. The DNA can also be obtained from mRNA by RT-PCR using a portion of the sequence described in SEQ ID NO: 1, 3, or 5 as a primer. Alternatively, the DNA may also be synthesized artificially with a commercially available DNA synthesizer.

Furthermore, DNA similar to the above DNA is also an example of the "substance regulating the expression and/or function of a zinc ion-requiring protein". The similar DNA can include DNA hybridizing to the sequence described in SEQ ID NO: 1, 3, or 5 under stringent condition and encoding the substance regulating the expression and/or function of a zinc ion-requiring protein, for example, a protein capable of regulating the activity of Snail. The DNA can be prepared as previously described above.

When each DNA described above is used, the DNA can be inserted into an appropriate vector and used. The DNA inserted into the vector is also one of the aspects of the present invention. As a vector to be used, a suitable vector can be selected depending on the purpose. Particularly, the vector can include mammal-derived vectors (e.g., pcDNA3 (manufactured by Invitrogen), pEGF-BOS (Nucleic Acids. Res., 18(17), p. 5322, 1990), pEF, pCDM8, pCXN), insect cell-derived vectors (e.g., "Bac-to-BAC baculovirus expression system" (manufactured by Invitrogen), pBacPAK8), plant-derived expression vectors (e.g., pMH1, pMH2), animal virus-derived vectors (e.g., pHSV, pMV, pAdexLcw), retrovirus-derived vectors (e.g., pZIPneo), yeast-derived vectors (e.g., "Pichia Expression Kit" (manufactured by Invitrogen), pNV11, SP-Q01), Bacillus subtilis-derived vectors (e.g., pPL608, pKTH50), Escherichia coli vectors (M13-type vectors, pUC-type vectors, pBR322, pBluescript, pCR-Script) and the like. In the present invention, it is preferred to use a vector capable of being expressed in a mammal cell and to use an expression vector. The vector can be introduced into a cell by a method selected from, for example, calcium phosphate method (Virology, Vol. 52, p. 456, 1973), DEAE dextran method, a method using cationic liposome DOTAP (manufactured by Roche Diagnostics), electroporation method (Nucleic Acids Res., Vol. 15, p. 1311, 1987), lipofection method (J. Clin. Biochem. Nutr., Vol. 7, p. 175, 1989), introduction method by viral infection (e.g., pMX, pMSCV; Sci. Am., p. 34, 1994), a particle gun and the like.

One example of the "substance regulating the expression and/or function of a zinc ion-requiring protein", for example, an agent suppressing the activity of Snail can include an antisense oligonucleotide targeting DNA or mRNA encoding LIV1. It is believed that the antisense oligonucleotide against the DNA encoding LIV1 prevents the expression of an endogenous LIV1 gene and negatively control the Snail activity. Such an antisense oligonucleotide can include an antisense oligonucleotide whose target sequence is DNA consisting of a sequence described in SEQ ID NO: 1, 3, or 5 or mRNA sequence generated from the DNA sequence. An example thereof can include an oligonucleotide described in SEQ ID NO: 7 or 8. In addition, any antisense oligonucleotide that can hybridize to any site in the DNA consisting of the above sequence described in SEQ ID NO: 1, 3, or 5, and the like, and can effectively inhibit LIV1 expression is included in the antisense oligonucleotide of the present invention. Such an antisense oligonucleotide is not necessarily completely complementary to the DNA consisting of the above sequence described in SEQ ID NO: 1, 3, or 5, or the corresponding mRNA.

The above described antisense oligonucleotide can be inserted into a suitable vector depending on the purposes and used. For example, for the purpose of applying it to a gene therapy, the vector may be selected appropriately from viral vectors such as retroviral, adenoviral and vaccinia viral vectors, and non-viral vectors such as cationic liposomes and ligand-DNA complexes, and the like. It can also be administered as a naked plasmid DNA (naked pDNA) together with a large volume of aqueous solutions without the use of carriers.

Another example of the "substance regulating the expression and/or function of a zinc ion-requiring protein", particularly the agent suppressing the activity of Snail can include double-stranded RNA having a sequence identical or similar to a portion of DNA encoding LIV1. A double-stranded RNA having a sequence identical or similar to a target gene sequence is capable of inducing RNA interference (RNAi) that inhibits the expression of the target gene. The RNAi refers to a phenomenon in which when double-stranded RNA (dsRNA) is introduced into a cell, an intracellular mRNA corresponding to the RNA sequence is specifically degraded and as a result is not expressed as a protein. The double-stranded RNA may be entirely composed of a region forming double strand or may have a partial region forming single strand (e.g., at both ends or either end) and the like. Thus, the double-stranded RNA of the present invention may also contain a region that is not double-stranded. An oligo RNA used in RNAi is often 10- to 100-bp RNA, and usually 19- to 23-bp RNA. The RNAi can be performed according to the descriptions of Nature, Vol. 391, p. 806, 1998, Proc. Natl. Acad. Sci. USA Vol. 95, p. 15502, 1998, Nature, Vol. 395, p. 854, 1998, Proc. Natl. Acad. Sci. USA Vol. 96, p. 5049, 1999, Cell, Vol. 95, p. 1017, 1998, Proc. Natl. Acad. Sci. USA Vol. 96, p. 1451, 1999, Proc. Natl. Acad. Sci. USA Vol. 95, p. 13959, 1998, Nature Cell Biol., Vol. 2, p. 70, 2000, and the like.

The "substance regulating the expression and/or function of a zinc ion transporter" is, for example, when the substance is LIV1, exemplified by those similar to the substances described above for the "substance regulating the expression and/or function of a zinc ion-requiring protein".

When the "zinc ion transporter" is other than LIV1, for example, hZnt-1, hZnt-2, hZnt-5, hZnt-6, hZnt-7, hZIP1, hZIP2, hZIP3, BAA92100, BAC04504, hLiv-1, hKE4, KIAA1265, KIAA0062, or hZip4, a variety of "substances regulating the expression and/or function of a zinc ion transporter" or "substances regulating the expression and/or function of a zinc ion-requiring protein" can be prepared in the same way as for LIV1, based on the known amino acid sequence or base sequence of the zinc ion transporter.

Information about the respective amino acid sequences and base sequences can be obtained from a variety of databases browsable in NCBI and the like.

The function controlling agent of the present invention for an antigen-presenting cell and the prophylactic and/or therapeutic drug of the present invention for a disease mediated by the immune system comprise, if desired, pharmaceutically acceptable excipients and additives, in addition to the above described series of active ingredients (zinc ion, zinc ionophore, zinc ion chelator, substance regulating the expression and/or function of a zinc ion-requiring protein, and substance regulating the expression and/or function of a zinc ion transporter). The pharmaceutically acceptable excipients and additives include carriers, binders, flavors, buffers, thickeners, coloring agents, stabilizers, emulsifiers, dispersants, suspending agents, preservatives and the like. Moreover, different types of "substances controlling the zinc ion concentration" can be used in combination as active ingredients.

The pharmaceutically acceptable carriers include, for example, magnesium carbonate, magnesium stearate, talc, sugar, lactose, pectin, dextrin, starch, gelatin, tragacanth, methylcellulose, sodium carboxymethylcellulose, low-melting wax, cocoa butter and the like.

Furthermore, tablets can be made, if necessary, into tablets coated with usual coatings, for example sugar-coated tablets, enteric-coated tablets, film-coated tablets, or two-layer tablets or multi-layer tablets. Powders can be prepared with pharmaceutically acceptable bases for powders. The bases include talc, lactose, starch and the like. Drops can be prepared together with aqueous or non-aqueous bases and one or more pharmaceutically acceptable dispersants, suspending agents, solubilizers, and the like. Capsules can be prepared by filling with a compound serving as an active ingredient together with a pharmaceutically acceptable carrier. The compound can be mixed with or without a pharmaceutically acceptable excipient and filled into capsules. Cachets can also be prepared in a similar way. When the present invention is prepared as a suppository, the suppository is prepared together with bases such as vegetable oil (e.g., castor oil, olive oil, peanut oil), mineral oil (e.g., petrolatum, white petrolatum), waxes, and partially or fully synthesized glycerin fatty acid ester, by an approach generally used.

Liquid formulations for injection include solutions, suspensions, emulsions and the like. Examples thereof include aqueous solutions, water-propylene glycol solutions and the like. The liquid formulations can also be produced in the form of a solution of polyethylene glycol and/or propylene glycol, which may contain water.

Liquid formulations suitable for oral administration can be produced by adding the compound serving as an active ingredient and, if necessary, a coloring agent, flavor, stabilizer, sweetening agent, solubilizer, thickener, or the like, to water. Alternatively, the liquid formulations suitable for oral administration can be produced by adding the compound and a dispersant to water to give a viscous liquid. Examples of the thickener include pharmaceutically acceptable natural or synthetic gum, resin, methylcellulose, sodium carboxymethylcellulose, known suspending agents and the like.

Agents for local administration include the liquid formation described above, and cream, aerosol, spray, powder, lotion, ointment and the like. The above described agents for local administration can be produced by mixing a compound serving as an active ingredient with pharmaceutically acceptable diluents and carriers. The ointment and cream can be prepared, for example, by adding thickeners and/or gelling agents to an aqueous or oily base. Examples of the base include water, liquid paraffin, vegetable oil and the like. Examples of the thickener include soft paraffin, aluminum stearate, cetostearyl alcohol, propylene glycol, polyethylene glycol, lanolin, hydrogenated lanolin, beeswax and the like. To the agent for local administration can be added, if necessary, a preservative such as methyl hydroxybenzoate, propyl hydroxybenzoate, chlorocresol, benzalkonium chloride and the like, or a bacterial growth inhibitor. The lotion can be prepared by adding one or more pharmaceutically acceptable stabilizers, suspending agents, emulsifiers, dispersants, thickeners, coloring agents, flavors, and the like, to an aqueous or oily base.

The thus-obtained function controlling agent of the present invention for an antigen-presenting cell and the prophylactic and/or therapeutic drug of the present invention for a disease mediated by the immune system are orally or parenterally administered.

When administered orally, they can be administered in a dosage form commonly used in the art. When administered parenterally, they can be administered in a dosage form such as agent for local administration (e.g., transdermal agent), agent for rectal administration, injectable, transnasal agent and the like.

Examples of the oral agent or agent for rectal administration include capsule, tablet, pill, powder, drop, cachet, suppository, liquid formulation and the like. Examples of the injectable include aseptic solutions or suspensions and the like. Examples of the agent for local administration include cream, ointment, lotion, transdermal agent (general patch agent or matrix agent) and the like.

The dosage and the number of administration may vary depending on the type of the substance capable of controlling the zinc ion concentration used, the condition, age and body weight of a patient, a dosage form and the like, and can be determined appropriately.

All of the "zinc ion", "zinc ion chelator", "substance regulating the expression and/or function of a zinc ion-requiring protein", and "substance regulating the expression and/or function of a zinc ion transporter" are capable of controlling the function of an antigen-presenting cell, specifically the expression of a molecule associated with antigen presentation, activation of an antigen-presenting cell by LPS and the like, cytokine production, antigen uptake, migration capability and the like in humans and other mammals such as cattle, horses, dogs, mice, and rats, and hence make it possible to prevent/treat a disease involved in the function of an antigen-presenting cell, specifically a disease mediated by the immune system. As the disease mediated by the immune system, autoimmune disease, allergic disease, cancer, graft rejection in organ transplant, immune response reductions due to infection or tumorigenesis, abnormal immune responses during vaccination (for example, adverse reactions) and the like can be mentioned. For example, the present invention is expected to be useful for treatment of SIRS (systemic inflammatory reaction syndrome), systemic lupus erythematosus, mixed connective tissue disease, rheumatoid arthritis, Sjogren's syndrome, rheumatic fever, Goodpasture syndrome, Basedow disease, Hashimoto disease, Addison disease, autoimmune hemolytic anemia, idiopathic thrombocytopenic purpura, myasthenia gravis, ulcerative colitis, Crohn disease, sympathetic ophthalmia, multiple sclerosis, psoriasis, anaphylactic shock, allergic rhinitis, allergic conjunctivitis, bronchial asthma, urticaria, atopic dermatitis and the like, for pre-transplant treatment or post-transplant administration for suppressing graft rejection in organ transplant, and for control of immune responses to cancer, and the like. As the cancer, cancers such as breast cancer, prostatic cancer, pancreatic cancer, gastric cancer, lung cancer, colorectal cancer (colic cancer, rectal cancer, anal cancer), esophageal cancer, duodenal cancer, cancer of head and neck (cancer of tongue, cancer of pharynx), brain tumor, schwannoma, non-small-cell lung cancer, small-cell lung cancer, liver cancer, kidney cancer, cancer of bile duct, uterine cancer (uterine body cancer, uterine cervical cancer), ovarian cancer, urinary bladder cancer, skin cancer, hemangioma, malignant lymphoma, malignant melanoma, thyroid cancer, bone tumor, hemangioma, angiofibroma, retinal sarcoma, penile cancer, solid cancers in children, Kaposi's sarcoma, AIDS-associated Kaposi's sarcoma, maxillary sinus tumor, fibrous histiocytoma, smooth muscle sarcoma, and rhabdomyosarcoma, and malignant tumors such as leukemia and the like can be mentioned.

By introducing a zinc ion into an antigen-presenting cell by means of a drug such as a zinc ionophore, it is possible to suppress the activation of an antigen-presenting cell by LPS and the like, and to suppress cytokine production, and even to control the balance between Th1 and Th2, and CTL and B cell activation. That is, the function controlling agent of the present invention for an antigen-presenting cell and the prophylactic and/or therapeutic drug of the present invention for a disease mediated by the immune system can be administered even to patients suffering from various diseases for which a therapeutic effect is observed when a zinc ion is introduced into an antigen-presenting cell.

Furthermore, based on the finding obtained by the present inventors that the function of an antigen-presenting cell can be controlled by controlling the zinc ion concentration, a compound capable of controlling the function of an antigen-presenting cell can be screened for by measuring the intracellular zinc ion concentration. This screening method is performed by, for example, the following steps:
1) a step for dividing antigen-presenting cells into two groups, and treating one group with a test compound (the remaining untreated group is used as a control);
2) a step for measuring the respective intracellular zinc ion concentrations for the treated cells and the untreated control cells; and
3) a step for selecting a compound that caused a significant change in zinc ion concentration compared to the zinc ion concentration in the control cell, and judging the compound as being capable of controlling the function of an antigen-presenting cell.

As the antigen-presenting cell, one of those described above is used. The antigen-presenting cell is preferably a dendritic cell. The cells are randomly divided into two groups, and one of which is treated with a test compound. The other is untreated and used as a control cell. Furthermore, a compound known to influence the zinc ion concentration, for example, the zinc ion chelator TPEN and the like, may be used as a positive control compound. The test compound may be a known compound or a novel compound to be developed in the future. It may be a low-molecular-weight compound or a high-molecular-weight compound. In this context, the low-molecular-weight compound is a compound having a molecular weight of less than approximately 3000, which includes, for example, an organic compound and a derivative thereof and an inorganic compound usually available as a pharmaceutical agent, and refers to a compound and a derivative thereof produced by making full use of organic synthesis or the like, a naturally occurring compound and a derivative thereof, small nucleic acid molecules such as promoter, a variety of metals and the like, and desirably an organic compound and a derivative thereof and a nucleic acid molecule available as a pharmaceutical agent. The high-molecular-weight compound is a compound having a molecular weight of not lower than approximately 3000, which includes, for example, protein, polynucleic acid, polysaccharide and combinations thereof, and is desirably a protein. These low-molecular-weight or high-molecular-weight compounds, if they are known, are commercially available or can be obtained by way of steps such as collection, production and purification according to the respective reported documents. These compounds may be naturally occurring or prepared by genetic engineering, or can also be obtained by semi-synthesis and the like.

The time period of the treatment of cells with the test compound is appropriately set depending on the types and concentrations of cells and test compounds to be used. When the positive control compound such as TPEN is used, the treatment can be performed using, as a guide, a change in the zinc ion concentration when treated with the control compound. The concentration of the test compound for treating cells is also appropriately set depending on the types of the cells and test compounds to be used, as well as the treatment period.

The measurement of the intracellular zinc ion concentration can be performed utilizing a method routinely practiced in the art or a method pursuant thereto. The concentration can be measured, for example, by direct measurement by atomic absorption method (flame method) or with a spectrophotofluorometer using a specific probe (e.g., fluorescent reagent).

### Examples

Hereinafter, although the present invention will be described in more detail with reference to Examples, these Examples do not limit the scope of the present invention by any means. All the publications cited throughout the present application are incorporated herein by reference. The reagents, apparatuses and materials used in the present invention are commercially available unless otherwise specified.

### Example 1

Dendritic cells, a type of antigen-presenting cells, were treated with N,N,N',N'-tetrakis(2-pyridylmethyl)ethylenediamine (TPEN), a zinc ion chelator, and examined for the expression of MHC class I molecule, class II molecule and CD86 molecule on the cell surface.

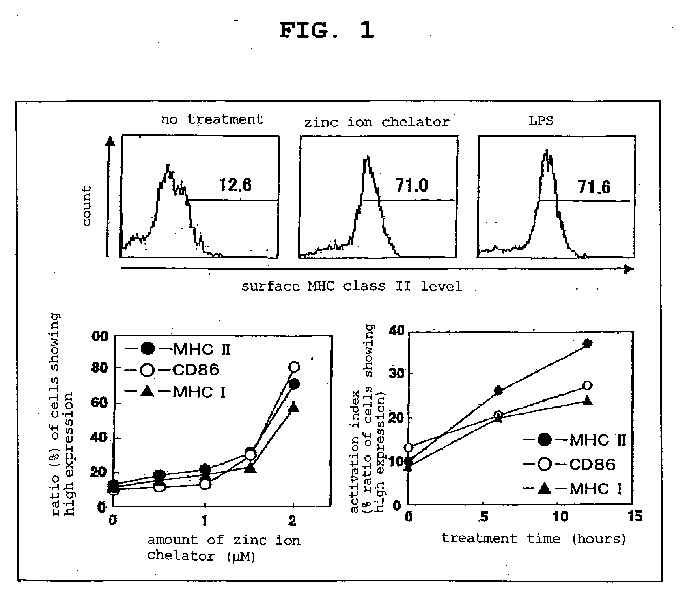

Bone marrow cells were obtained from a C57BL/6 mouse (obtained from Clea Japan) and subjected to cell culture using an RPMI medium (SIGMA, R8758) supplemented with 10% FCS (EQUITECH-BIO Inc., #SFB-30-1388) and granulocyte macrophage colony stimulating factor (GM-CSF) in a 5% CO₂ environment at 37°C for 6 days to induce the differentiation of bone marrow-derived dendritic cells (BMDC). To this BMDC, TPEN (SIGMA, P4413) was added for 6 hours to 12 hours to obtain concentrations of 0-2 µM in the medium. Also prepared was a group in which lipopolysaccharide (LPS; 100 ng/mL; SIGMA, L2637, E. Coli 055:B5) was added for control of antigen-presenting cell activation. The MHC class I, class II and CD86 molecules that became expressed on the cell surface after 12 hours (or 6 hours) were evaluated by FACS analysis. The results are shown in Figure 1. Each numerical value in Figure 1 shows the percentage of cells highly expressing each molecule. When the cells were treated with the zinc ion chelator, the expression of MHC class II molecule on BMDC was enhanced as with LPS. This expression enhancement was observed with drug dose dependency (treatment time of 12 hours) and treatment time dependency (treatment concentration of 1.5 µM).

### Example 2

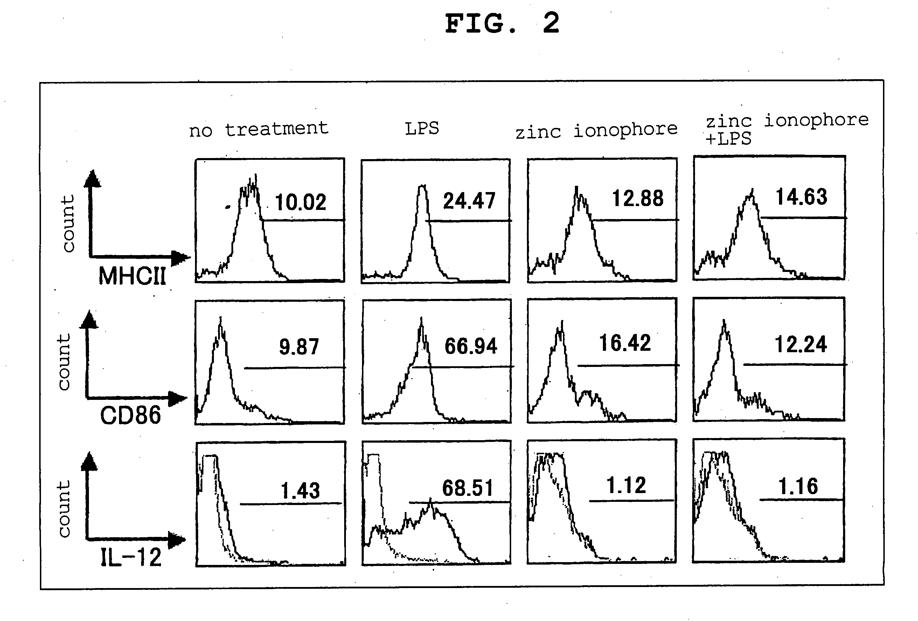

The zinc ionophore pyrithione (5 µM; Molecular probes, p-24193) and Zn²⁺ (ZnSO₄) were added to BMDC prepared in the same manner as Example 1 to obtain a final concentration of 5 µM in the culture broth, and the BMDC was further stimulated with LPS (100 ng/mL). The MHC class II and CD86 molecules that became expressed on the cell surface after 6 hours were evaluated by FACS analysis. The results are shown in Figure 2. Each numerical value in Figure 2 shows the percentage of cells highly expressing each molecule. At the same time, IL-12 production by the BMDC was detected by the intracellular staining method using an anti-IL-12p40 antibody (BD.Bioscience Pharmingen, #554479) and an isotype control antibody (BD.Bioscience Pharmingen, #553925) after fixation with 4% para-formaldehyde. Each numerical value in the figure shows the percentage of cells that produced IL-12.

No expression enhancement of MHC II and CD86 molecules on the cell surface or IL-12 cytokine production that accompany LPS-stimulated BMDC activation was observed in the presence of the zinc ionophore.

In the same experiment (in vitro system) as Examples 1 and 2, dendritic cell activation or inactivation was observed by a treatment with 1-10 µM zinc ion or TPEN.

### Example 3

Using a retrovirus vector that expresses mouse Liv1 as the substance that controls the zinc ion concentration, its effect on LPS-stimulated expression enhancement of MHC class II molecule was examined.

A clone comprising the mouse Liv1 gene (mLiv1, gene sequence: SEQ ID NO:5, amino acid sequence: SEQ ID NO:6) supplied by RIKEN (mfj04623) was cleaved with the restriction endonucleases XhoI and NotI, and the resulting mLiv1 gene was recovered and inserted in the pMX-IRES-GFP retrovirus vector prepared and supplied by Professor Toshio Kitamura at the University of Tokyo Institute of Medical Science. This pMX-IRES-GFP-mLiv1 vector was introduced into Phoenix cells supplied by Dr. Garry P. Nolan at Stanford University of USA using lipofectamine (Invitrogen) to express the virus. After 24 hours to 48 hours, the culture supernatant containing this virus was recovered, GM-CSF [a GM-CSF-producing cell line (GM-CSF/CHO) was cultured, and the culture supernatant was added to the medium to obtain a concentration of 0.3%], FCS (10%), and protamine sulfate (SIGMA; 2 µg/mL) were added, and the mixture was used as the virus-infection liquid. BMDC prepared from the bone marrow of a C57BL/6 mouse (Clea Japan) were infected by exchanging the medium with the virus-infection liquid on Day 2 and Day 4. On Day 6, the liquid was exchanged with a virus-free medium, and the BMDC was further stimulated with LPS (SIGMA; 100 ng/mL). Six hours after the stimulation, the cells were recovered, and the expression level of MHC class II molecule on the cell surface was examined by FACS. Here, a group of cells not stimulated with LPS were handled as controls, and GFP-positive cells as cells showing forced expression of mLiv1.

It was found that expression enhancement of MHC class II molecule by LPS stimulation was suppressed in cells showing forced expression of mLiv1.

### Free-text of Sequence Listing

SEQ ID No: 7: Antisense sequence
SEQ ID No: 8: Antisense sequence

### Industrial Applicability

In an antigen-presenting cell, by controlling the zinc ion concentration, or by regulating the expression or function of a zinc ion-requiring protein and a zinc ion transporter, the expression of a molecule associated with antigen presentation (for example, MHC class I, class II, CD86 molecule and the like) can be enhanced. Furthermore, by introducing a zinc ion into an antigen-presenting cell by means of a drug such as a zinc ionophore, activation of an antigen-presenting cell and cytokine production by lipopolysaccharide (LPS) and the like can be suppressed. Therefore, simply by regulating the intracellular level of zinc essentially present in the body, without using conventional immunopotentiating molecules, immunosuppressants and the like such as microbial constituents and synthetic steroids, it is possible, for example, to enhance or suppress the immune system via activation of an antigen-presenting cell such as a dendritic cell.

This application is based on a patent application No. 2005-023200 filed in Japan on January 31, 2005, the contents of which are incorporated in full herein by this reference.

## Claims

1. An agent for controlling the function of an antigen-presenting cell, comprising a substance capable of controlling the intracellular zinc ion concentration as the active ingredient.

2. The agent of claim 1, wherein the substance capable of controlling the zinc ion concentration is a zinc ion.

3. The agent of claim 2, wherein the zinc ion is introduced into a cell through a zinc ionophore.

4. The agent of claim 1, wherein the substance capable of controlling the zinc ion concentration is a zinc ion chelator.

5. The agent of claim 4, wherein the zinc ion chelator is at least one selected from the group consisting of 2,3-dimercapto-1-propanesulfonic acid (DMPS), N,N,N',N'-tetrakis(2-pyridylmethyl)ethylenediamine (TPEN), ethylenediamine (EDTA) and N-(6-methoxy-8-quinolyl)-p-toluenesulfonamide (TSQ).

6. The agent of claim 1, wherein the substance capable of controlling the zinc ion concentration is a substance regulating the expression and/or function of a zinc ion-requiring protein.

7. The agent of claim 6, wherein the zinc ion-requiring protein is at least one selected from the group consisting of Raf-1, PKCα, GEF-H1, HDAC, SQSTM1, ubiquitin-protein ligase E3, ubiquitin-conjugating enzyme E2, metallothionein, phosphatase, Snail, TRAF6, Paxillin, Zyxin and Jade-1.

8. The agent of claim 1, wherein the substance capable of controlling the zinc ion concentration is a substance regulating the expression and/or function of a zinc ion transporter.

9. The agent of claim 8, wherein the zinc ion transporter is at least one selected from the group consisting of human ZIPs including LIV family and human CDFs.

10. The agent of any one of claims 1 to 9, wherein the antigen-presenting cell is at least one selected from the group consisting of a dendritic cell, a macrophage, a Langerhans' cell, a B cell, a thymic epithelial cell, a synovial cell, a vascular endothelial cell and a keratinocyte.

11. The agent of claim 10, wherein the antigen-presenting cell is a dendritic cell.

12. A prophylactic and/or therapeutic drug for a disease mediated by the immune system, comprising the function controlling agent of any one of claims 1 to 11.

13. The prophylactic and/or therapeutic drug of claim 12, wherein the disease mediated by the immune system is at least one selected from the group consisting of autoimmune disease, allergic disease, graft rejection in organ transplant, an immune response reduction due to infection or tumorigenesis, and an abnormal immune response during vaccination.

14. A prophylactic and/or therapeutic drug for a disease mediated by the immune system, comprising a substance capable of controlling the intracellular zinc ion concentration as the active ingredient.

15. The prophylactic and/or therapeutic drug of claim 14, wherein the substance capable of controlling the zinc ion concentration is a zinc ion.

16. The prophylactic and/or therapeutic drug of claim 15, wherein the zinc ion is introduced into a cell through a zinc ionophore.

17. The prophylactic and/or therapeutic drug of claim 14, wherein the substance capable of controlling the zinc ion concentration is a zinc ion chelator.

18. The prophylactic and/or therapeutic drug of claim 17, wherein the zinc ion chelator is at least one selected from the group consisting of 2,3-dimercapto-1-propanesulfonic acid (DMPS), N,N,N',N'-tetrakis(2-pyridylmethyl)ethylenediamine (TPEN), ethylenediamine (EDTA) and N-(6-methoxy-8-quinolyl)-p-toluenesulfonamide (TSQ).

19. The prophylactic and/or therapeutic drug of claim 14, wherein the substance capable of controlling the zinc ion concentration is a substance regulating the expression and/or function of a zinc ion-requiring protein.

20. The prophylactic and/or therapeutic drug of claim 19, wherein the zinc ion-requiring protein is at least one selected from the group consisting of Raf-1, PKCα, GEF-H1, HDAC, SQSTM1, ubiquitin-protein ligase E3, ubiquitin-conjugating enzyme E2, metallothionein, phosphatase, Snail, TRAF6, Paxillin, Zyxin and Jade-1.

21. The prophylactic and/or therapeutic drug of claim 14, wherein the substance capable of controlling the zinc ion concentration is a substance regulating the expression and/or function of a zinc ion transporter.

22. The prophylactic and/or therapeutic drug of claim 21, 5 wherein the zinc ion transporter is at least one selected from the group consisting of human ZIPs including LIV family and human CDFs.

23. The prophylactic and/or therapeutic drug of any one of claims 14 to 22, wherein the disease mediated by the immune system is at least one selected from the group consisting of autoimmune disease, allergic disease, graft rejection in organ transplant, an immune response reduction due to infection or tumorigenesis and an abnormal immune response during vaccination.

24. The prophylactic and/or therapeutic drug of any one of claims 14 to 23, wherein the antigen-presenting cell is at least one selected from the group consisting of a dendritic cell, a macrophage, a Langerhans' cell, a B cell, a thymic epithelial cell, a synovial cell, a vascular endothelial cell and a keratinocyte.

25. The prophylactic and/or therapeutic drug of claim 24, wherein the antigen-presenting cell is a dendritic cell.

26. A method of controlling the function of an antigen-presenting cell, comprising controlling the intracellular zinc ion concentration in vitro.

27. The method of claim 26, wherein the zinc ion concentration is controlled by introducing a zinc ion into a cell.

28. The method of claim 27, wherein the zinc ion is introduced into the cell through a zinc ionophore.

29. The method of claim 26, wherein the zinc ion concentration is controlled by a zinc ion chelator.

30. The method of claim 29, wherein the zinc ion chelator is at least one selected from the group consisting of 2,3-dimercapto-1-propanesulfonic acid (DMPS), N,N,N',N'-tetrakis(2-pyridylmethyl)ethylenediamine (TPEN), ethylenediamine (EDTA) and N-(6-methoxy-8-quinolyl)-p-toluenesulfonamide (TSQ).

31. The method of claim 26, wherein the zinc ion concentration is controlled by regulating the expression and/or function of a zinc ion-requiring protein.

32. The method of claim 31, wherein the zinc ion-requiring protein is at least one selected from the group consisting of Raf-1, PKCα, GEF-H1, HDAC, SQSTM1, ubiquitin-protein ligase E3, ubiquitin-conjugating enzyme E2, metallothionein, phosphatase, Snail, TRAF6, Paxillin, Zyxin and Jade-1.

33. The method of claim 26, wherein the zinc ion concentration is controlled by regulating the expression and/or function of a zinc ion transporter.

34. The method of claim 33, wherein the zinc ion transporter is at least one selected from the group consisting of human ZIPs including LIV family and human CDFs.

35. The method of any one of claims 26 to 34, wherein the antigen-presenting cell is at least one selected from the group consisting of a dendritic cell, a macrophage, a Langerhans' cell, a B cell, a thymic epithelial cell, a synovial cell, a vascular endothelial cell and a keratinocyte.

36. A screening method for a compound capable of controlling the function of an antigen-presenting cell, comprising measuring the intracellular zinc ion concentration.

37. The method of claim 36, wherein the antigen-presenting cell is at least one selected from the group consisting of a dendritic cell, a macrophage, a Langerhans' cell, a B cell, a thymic epithelial cell, a synovial cell, a vascular endothelial cell and a keratinocyte.

38. The method of claim 37, wherein the antigen-presenting cell is a dendritic cell.

39. A use of a substance capable of controlling the intracellular zinc ion concentration, for producing a function controlling agent for an antigen-presenting cell.

40. A use of the function controlling agent of any one of claims 1 to 11, for producing a prophylactic and/or therapeutic drug for a disease mediated by the immune system.

41. A use of a substance capable of controlling the intracellular zinc ion concentration, for producing a prophylactic and/or therapeutic drug for a disease mediated by the immune system.
